# EUROPEAN PATENT SPECIFICATION

(11) **EP 2 970 886 B1**
(45) Date of publication and mention of the grant of the patent: **23.05.2018**
(21) Application number: 14779898.7
(22) Date of filing: 12.03.2014
(51) Int. Cl.: C12N 5/00, C12N 5/02, C12N 5/0783

(54) **METHODS AND COMPOSITIONS FOR MODIFICATION OF HLA**
VERFAHREN UND ZUSAMMENSETZUNGEN ZUR HLA-MODIFIKATION
PROCÉDÉS ET COMPOSITIONS POUR MODIFICATION DE HLA

(30) Priority: 12.03.2013 US 201361777627 P
(43) Date of publication of application: 20.01.2016
(73) Proprietor: Sangamo Therapeutics, Inc., Richmond, CA 94804 (US); Board of Regents, The University of Texas System, Austin, TX 78701 (US)
(72) Inventor: GREGORY, Philip D., Richmond California 94804 (US); COOPER, Laurence J.N., Austin Texas 78701 (US); TORIKAI, Hiroki, Austin Texas 78701 (US)
(74) Representative: Brasnett, Adrian Hugh
(86) International application number: PCT/US2014/024660
(87) International publication number: WO 2014/165177

(56) References cited:
- WO-A1-2013/074916
- US-A1- 2010 291 048
- Holmes MC, Torikai H, Reik A, Yuen C, Zhou Y et al.: "Disruption of HLA expression to enable allogeneic cellsto escape immune recognition", Human gene Therapy, vol. 22 27 October 2011 (2011-10-27), page A25, XP002759588, DOI: 10.1089/hum.2011.2525 Retrieved from the Internet: URL:http://online.liebertpub.com/doi/pdf/1 0.1089/hum.2011.2525 [retrieved on 2016-07-06]
- HIROKI TORIKAI ET AL: "Toward eliminating HLA class I expression to generate universal cells from allogeneic donors", BLOOD, AMERICAN SOCIETY OF HEMATOLOGY, US, vol. 122, no. 8, 22 August 2013 (2013-08-22), pages 1341-1349, XP002719612, ISSN: 0006-4971, DOI: 10.1182/BLOOD-2013-03-478255 [retrieved on 2013-06-05]
- PETER PARHAM: "MHC class I molecules and kirs in human history, health and survival", THE JOURNAL OF IMMUNOLOGY, vol. 5, no. 3, 18 February 2005 (2005-02-18), pages 201-214, XP55286333, GB ISSN: 1474-1733, DOI: 10.1038/nri1570
- JAMIESON A C ET AL: "Drug discovery with engineered zinc-finger proteins", NATURE REVIEWS. DRUG DISCOVERY, NATURE PUBLISHING GROUP, GB, vol. 2, no. 5, 1 May 2003 (2003-05-01), pages 361-368, XP002342391, ISSN: 1474-1784, DOI: 10.1038/NRD1087
- L'HARIDON MYRIAM ET AL: "Transcriptional regulation of the MHC class I HLA-A11 promoter by the zinc finger protein ZFX", NUCLEIC ACIDS RESEARCH, INFORMATION RETRIEVAL LTD, GB, vol. 24, no. 10, 1 January 1996 (1996-01-01), pages 1928-1935, XP002719610, ISSN: 0305-1048
- TORIKAI, H ET AL.: 'HLA And TCR Knockout By Zinc Finger Nucleases: Toward 'Off-The-Shelf Allogenic T- Cell Therapy For CD 19+ Malignancies' 53RD ASH ANNUAL MEETING AND EXPOSITION. 10 December 2011, XP055283845 Retrieved from the Internet: <URL:https://ash.confex.com/ash/2010/webpro gram/Paper32101.html> [retrieved on 2014-07-14]
- BUKUR, J ET AL.: 'The Role Of Classical And Non-Classical HLA Class I Antigens In Human Tumors.' SEMIN CANCER BIOL. vol. 22, no. 4, August 2012, pages 350 - 358, XP028429657

## Description

### TECHNICAL FIELD

The present disclosure is in the fields of gene expression, genome engineering and gene therapy.

### BACKGROUND

MHC antigens were first characterized as proteins that played a major role in transplantation reactions. Rejection is mediated by T cells reacting to the histocompatibility antigens on the surface of implanted tissues, and the largest group of these antigens is the major histocompatibility antigens (MHC). These proteins are expressed on the surface of all higher vertebrates and are called H-2 antigens in mice (for histocompatibility-2 antigens) and HLA antigens (for human leukocyte antigens) in human cells.

The MHC proteins serve a vital role in T cell stimulation. Antigen presenting cells (often dendritic cells) display peptides that are the degradation products of foreign proteins on the cell surface on the MHC. In the presence of a co-stimulatory signal, the T cell becomes activated, and will act on a target cell that also displays that same peptide/MHC complex. For example, a stimulated T helper cell will target a macrophage displaying an antigen in conjunction with its MHC, or a cytotoxic T cell (CTL) will act on a virally infected cell displaying foreign viral peptides.

MHC proteins are of two classes, I and II. The class I MHC proteins are heterodimers of two proteins, the α chain, which is a transmembrane protein encoded by the MHC1 class I genes, and the β2 microblogulin chain, which is a small extracellular protein that is encoded by a gene that does not lie within the MHC gene cluster. The α chain folds into three globular domains and when the β2 microglobulin chain is associated, the globular structure complex is similar to an antibody complex. The foreign peptides are presented on the two most N-terminal domains which are also the most variable. Class II MHC proteins are also heterodimers, but the heterodimers comprise two transmembrane proteins encoded by genes within the MHC complex. The class I MHC:antigen complex interacts with cytotoxic T cells while the class II MHC presents antigens to helper T cells. In addition, class I MHC proteins tend to be expressed in nearly all nucleated cells and platelets (and red blood cells in mice) while class II MHC protein are more selectively expressed. Typically, class II MHC proteins are expressed on B cells, some macrophage and monocytes, Langerhans cells, and dendritic cells.

The class IHLA gene cluster in humans comprises three major loci, B, C and A, as well as several minor loci. The class II HLA cluster also comprises three major loci, DP, DQ and DR, and both the class I and class II gene clusters are polymorphic, in that there are several different alleles of both the class I and II genes within the population. There are also several accessory proteins that play a role in HLA functioning as well. The Tap1 and Tap2 subunits are parts of the TAP transporter complex that is essential in loading peptide antigens on to the class I HLA complexes, and the LMP2 and LMP7 proteosome subunits play roles in the proteolytic degradation of antigens into peptides for display on the HLA. Reduction in LMP7 has been shown to reduce the amount of MHC class I at the cell surface, perhaps through a lack of stabilization (see Fehling et al (1999) Science 265:1234-1237). In addition to TAP and LMP, there is the tapasin gene, whose product forms a bridge between the TAP complex and the HLA class I chains and enhances peptide loading. Reduction in tapasin results in cells with impaired MHC class I assembly, reduced cell surface expression of the MHC class I and impaired immune responses (see Grandea et al (2000) Immunity 13:213-222 and Garbi et al (2000) Nat Immunol 1:234-238).

Regulation of class I expression is generally at the transcriptional level, and several stimuli such as viral infection etc. can cause a change in transcription. The class I genes are down-regulated in some specific tissues, and the source of this down-regulation seems to be within the promoter and 3' intergenic sequences (see Cohen et al (2009) PLos ONE 4(8): e6748). There is also evidence that microRNAs are capable of regulating some class I MHC genes (see Zhu et al, (2010) Am. J. Obstet Gynecol 202(6):592).

Regulation of class II MHC expression is dependent upon the activity of the MHCII enhanceosome complex. The enhanceosome components (one of the most highly studied components of the enhanceosome complex is the RFX5 gene product (see Villard et al (2000) MCB 20(10): 3364-3376)) are nearly universally expressed and expression of these components does not seem to control the tissue specific expression of MHC class II genes or their IFN-γ induced up-regulation. Instead, it appears that a protein known as CIITA (class II transactivator) which is a non-DNA binding protein, serves as a master control factor for MCHII expression. In contrast to the other enhanceosome members, CIITA does exhibit tissue specific expression, is up-regulated by IFN-γ, and has been shown to be inhibited by several bacteria and viruses which can cause a down regulation of MHC class II expression (thought to be part of a bacterial attempt to evade immune surveillance (see LeibundGut-Landmann et al (2004) Eur. J. Immunol 34:1513-1525)).

Regulation of the class I or II genes can be disrupted in the presence of some tumors and such disruption can have consequences on the prognosis of the patients. For example, in some melanomas, an observed reduction in Tap 1, Tap 2 and HLA class I antigens was found to be more common in metastatic melanomas (P<0.05) than in primary tumors (see, Kagashita et al (1999) Am Jour of Pathol 154(3):745-754).

In humans, susceptibility to several diseases is suspected to be tied to HLA haplotype. These diseases include Addison's disease, ankylosing spondylitis, Behçet's disease, Buerger's disease, celiac disease, chronic active hepatitis, Graves' disease, juvenile rheumatoid arthritis, psoriasis, psoriatic arthritis, rheumatoid arthritis, Sjögren syndrome, and lupus erythematosus, among others.

HLA also plays a major role in transplant rejection. The acute phase of transplant rejection can occur within about 1-3 weeks and usually involves the action of host T lymphocytes on donor tissues due to sensitization of the host system to the donor class I and class II HLA molecules. In most cases, the triggering antigens are the class I HLAs. For best success, donors are typed for HLA and matched to the patient recipient as completely as possible. But donation even between family members, which can share a high percentage of HLA identity, is still often not successful. Thus, in order to preserve the graft tissue within the recipient, the patient often must be subjected to profound immunosuppressive therapy to prevent rejection. Such therapy can lead to complications and significant morbidities due to opportunistic infections that the patient may have difficulty overcoming.

Cell therapy is a specialized type of transplant wherein cells of a certain type (*e.g.* T cells reactive to a tumor antigen or B cells) are given to a recipient. Cell therapy can be done with cells that are either autologous (derived from the recipient) or allogenic (derived from a donor) and the cells may be immature cells such as stem cells, or completely mature and functional cells such as T cells. In fact, in some diseases such certain cancers, T cells may be manipulated *ex vivo* to increase their avidity for certain tumor antigens, expanded and then introduced into the patient suffering from that cancer type in an attempt to eradicate the tumor. This is particularly useful when the endogenous T cell response is suppressed by the tumor itself. However, the same caveats apply for cell therapy as apply for more well-known solid organ transplants in regards to rejection. Donor T cells express class I HLA antigens and thus are capable of eliciting a rejection response from the recipient's endogenous immune system.

U.S. Patent Publication No. 2012/0060230 describes specific zinc finger protein regulators of classic HLA genes such as HLA-A, HLA-B, HLA-C. These regulators can be used to make cells (*e.g*., stem cells) that do not express one or more classic HLA genes and, accordingly, can be used for autologous transplants. However, the loss of classic HLA expression may render the genetically modified cells targets for natural killed (NK)-cell mediated cytotoxicity based on loss of ligands for KIR. *See, e.g.,* Parham et al. (2005) Nat Rev Immunol. 5(3):201-214.

Holmes et al. (2011) Human Gene Therapy 22:A25 and Torikai et al. (poster presentation at the 53rd ASH Annual Meeting and Exposition, 10-13 December 2011, San Diego, CA) describe the disruption of HLA-A gene expression in T-cells using zinc finger nucleases.

Thus, there remains a need for compositions and methods for developing cells that lack some or all classic HLA expression but which cells are not targeted by NK cells for lysis.

### SUMMARY

The present invention provides an isolated Natural Killer (NK) cell comprising one or more non-classic class I human leukocyte antigen (HLA) proteins and further wherein at least one classic endogenous HLA gene within the cell is inactivated by a zinc finger nuclease, as defined in the claims.

The present invention also provides a pharmaceutical composition comprising the NK cell and a pharmaceutical composition for use, as defined in the claims.

The present invention also provides a method of generating an isolated Natural Killer (NK) cell in which one or more classic HLA genes are inactivated and in which one or more non-classic HLA proteins are present within the cell, the method comprising; a) cleaving an endogenous HLA gene or HLA regulator gene in said cell with a zinc finger nuclease, such that the HLA or HLA regulator gene is inactivated, and; b) introducing a nucleic acid encoding a non-classic HLA molecule into the cell, as defined in the claims.

The present disclosure also sets out ("provides") a number of compositions and methods as discussed herein below. The invention is however defined by the accompanying claims.

Disclosed herein are methods and compositions for modifying HLA expression. In particular, provided herein are methods and compositions for modulating expression of an HLA gene so as to treat HLA-related disorders, for example human disorders related to HLA haplotype of the individual. Additionally, provided herein are methods and compositions for deleting (inactivating) or repressing an HLA gene to produce an HLA null cell, cell fragment (*e.g.* platelet), tissue or whole organism, for example a cell that does not express one or more classic HLA genes. Additionally, these methods and compositions may be used to create a cell, cell fragment, tissue or organism that is null for just one classic HLA gene, or more than one classic HLA gene, or is completely null for all classic HLA genes. In certain embodiments, the classic HLA null cells or tissues are human cells or tissues that are advantageous for use in transplants.

Thus, in one aspect, described herein are cells in which one or more classic HLA genes are inactivated and in which one or more non-classic HLA proteins (*e.g*., HLA-E, HLA-F, HLA-G) are present within the cell. The non-classical class I HLA molecules may be expressed (over-expressed) from endogenous genes, may be added to the cell and/or may be expressed by genetic modification of the cell (*e.g*., stable or transient transfection of polynucleotides expressing the one or more non-classical HLA molecules). In certain embodiments, the non-classical HLA molecules comprise HLA-E and/or HLA-G.

The modified cells may be a lymphoid cell (*e.g*., natural killer (NK) cell, a T-cell, a B-cell), a myeloid cell (*e.g*., monocyte, neutrophil, dendritic cell, macrophage, basophil, mast cell); a stem cell(*e.g*., an induced pluripotent stem cell (iPSC), an embryonic stem cell (*e.g*., human ES), a mesenchymal stem cell (MSC), a hematopoietic stem cell (HSC) or a neuronal stem cell) or a fragment of a cell (*e.g.,* platelet). The stem cells may be totitpotent or pluripotent (*e.g*., partially differentiated such as an HSC that is a pluripotent myeloid or lymphoid stem cell). In some embodiments, the modified cells in which expression of more than one classic HLA gene have been altered, expression of one or more non-classic HLA(s) is also altered. In other embodiments, the invention provides methods for producing stem cells that have a null phenotype for one or more or all classic HLA genes. Any of the modified stem cells described herein (modified at the HLA locus/loci) may then be differentiated to generate a differentiated (*in vivo* or *in vitro*) cell descended from a stem cell as described herein.

In other embodiments, described herein are methods of reducing natural killer (NK) cell lysis of a cell lacking one or more classic HLA genes (*e.g*., via nuclease-mediated inactivation of the one or more genes), the method comprising providing a cell as described herein (*e.g.,* a cell in which classic HLA gene(s) is(are) inactivated and in in which one or more non-classic HLA molecules are present), thereby reducing NK mediated cell lysis.

In another aspect, the compositions (modified cells) and methods described herein can be used, for example, in the treatment or prevention or amelioration of any HLA-related disorder (*i.e.,* related to HLA haplotype). The methods typically comprise (a) cleaving an endogenous HLA gene or HLA regulator gene in an isolated cell (*e.g.,* T-cell or lymphocyte) using a nuclease (*e.g.,* ZFN or TALEN) or nuclease system such as CRISPR/Cas with an engineered crRNA/tracr RNA such that the HLA or HLA regulator gene is inactivated; (b) introducing a non-classic HLA molecule into the cell; and (c) introducing the cell into the subject, thereby treating or preventing an HLA-related disorder. In certain embodiments, the HLA-related disorder is graft-versus-host disease (GVHD). The nuclease(s) can be introduced as mRNA, in protein form and/or as a DNA sequence encoding the nuclease(s). Likewise the non-classic HLA molecules (*e.g*., HLA-E and/or HLA-G) may be introduced as mRNA, in protein form and/or as a DNA sequence encoding the molecules. In certain embodiments, the isolated cell introduced into the subject further comprises additional genomic modification, for example, an integrated exogenous sequence (into the cleaved HLA or HLA regulatory gene or a different gene, for example a safe harbor gene) and/or inactivation (*e.g*., nuclease-mediated) of additional genes, for example one or more TCR genes. The exogenous sequence may be introduced via a vector (*e.g.* Ad, AAV, LV), or by using a technique such as electroporation. In some aspects, the composition may comprise isolated cell fragments and/or differentiated (partially or fully) cells.

Also provided are pharmaceutical compositions comprising the modified cells as described herein (*e.g*., stem cells with inactivated classic HLA gene(s) and which express non-classic HLA gene(s)). In certain embodiments, the pharmaceutical compositions further comprise one or more pharmaceutically acceptable excipients. Such pharmaceutical compositions may be used prophylactically or therapeutically and may comprise iPSCs, hES, MSCs, HSCs or combinations and/or derivatives thereof. In other embodiments, cells, cell fragments (*e.g*., platelets) or tissues derived from such modified stem cells are provided such that such tissues are modified in the HLA loci as desired. In some aspects, such cells are partially differentiated (*e.g*. hematopoietic stem cells) while in others fully differentiated cells are provided (*e.g*. lymphocytes or megakarocytes) while in still others, fragments of differentiated cells are provided. In other embodiments, stem cells, and/or their differentiated progeny are provided that contain an altered HLA or HLA regulator gene or genes, and they also can contain an additional genetic modification including a deletion, alteration or insertion of a donor DNA at another locus of interest.

In some embodiments, cells as described herein may be mature cells such as CD4+ T cells or NK cells. In some aspects, the mature cells may be used for cell therapy, for example, for a T cell transplant. In other embodiments, the cells for use in T cell transplant contain another gene modification of interest. In one aspect, the T cells contain an inserted chimeric antigen receptor (CAR) specific for a cancer marker. In a further aspect, the inserted CAR is specific for the CD19 marker characteristic of B cell malignancies. Such cells would be useful in a therapeutic composition for treating patients without having to match HLA, and so would be able to be used as an "off-the-shelf' therapeutic for any patient in need thereof. In some aspects, cells in which genes encoding the T-cell receptors (TCR) genes (*e.g*., TCRα and/or TCRβ chains) have been manipulated or in which genes encoding TCR chains with desired specificity and affinity have been introduced are provided. In other embodiments, HLA modified platelets are provided for therapeutic use in treatment of disorders such as thromobytopenia or other bleeding disorders.

Any of the methods described herein can be practiced *in vitro, in vivo* and/or *ex vivo.* In certain embodiments, the methods are practiced *ex vivo,* for example to modify stem cells, T-cells or NK cells prior to use for treating a subject in need thereof.

These and other aspects will be readily apparent to the skilled artisan in light of disclosure as a whole.

### BRIEF DESCRIPTION OF THE DRAWINGS

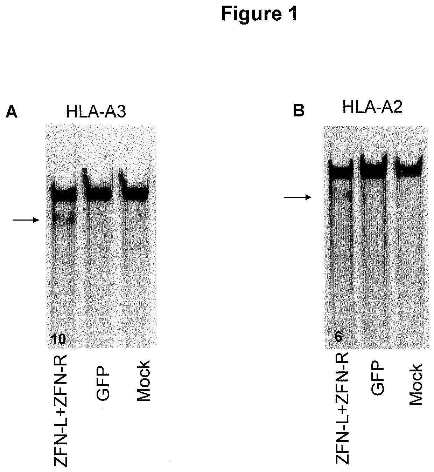

**Figure 1****, panels A and B,** shows levels of HLA-A3 (Figure 1A) and HLA-A2 (Figure 1B) genetic disruption assessed by the Surveyor™ nuclease assay. The lower (fast-moving) bands (arrows) are digestion products indicating ZFN-mediated gene modification. The numbers at the bottom of the lanes indicate the percentage of modified HLA-A alleles based on densitometry. DNA from mock transfected cells and cells transfected with a GFP expression vector was used for negative controls.

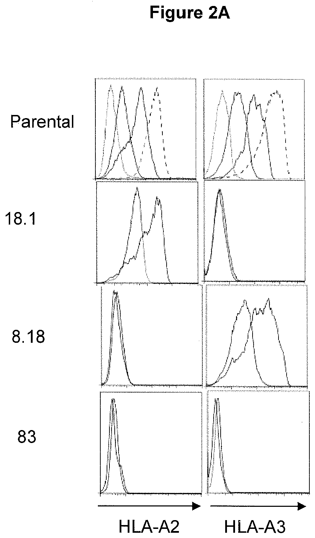

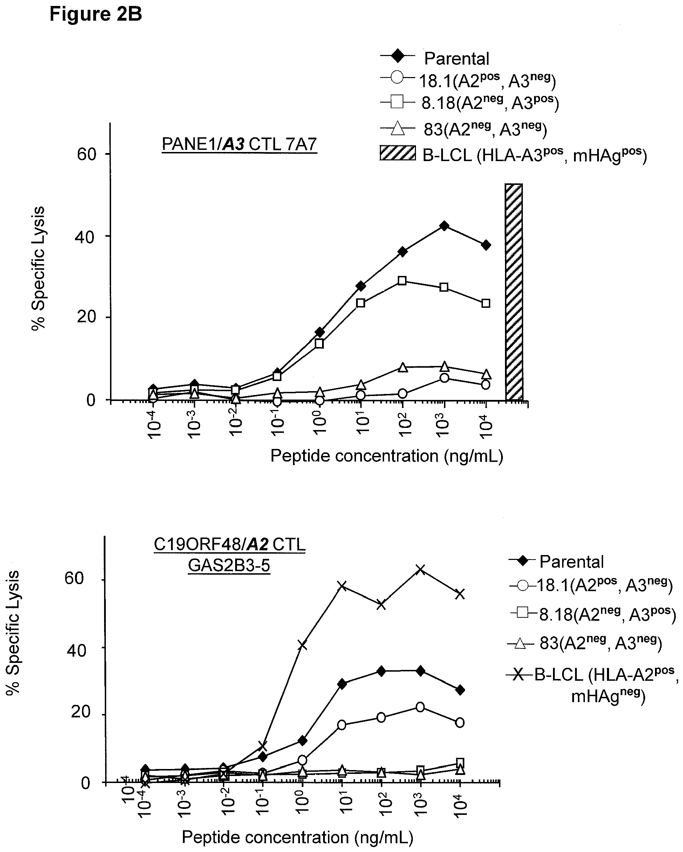

**Figure 2****, panels A and B,** show isolation of HLA-A^{neg} HEK293. Figure 2A shows loss of HLA-A2 and HLA-A3 protein expression. Flow cytometry analysis of HLA-A2 and HLA-A3 expression on parental HEK293 cells and three derived genetically modified clones with loss of HLA-A (numbered 18.1, 8.18, 83). Dotted lines represent isotype (HLA-A2) or SA-PE (HLA-A3) controls, solid line represents HLA-A expression without IFN-γ and TNF-α, and filled lines represent HLA-A expression after culturing with 600 IU/mL of IFN-γ and 10 ng/mL of TNF-α for 48 hours. Dashed lines in the parental column represent HLA-A2 or HLA-A3 expression on EBV-LCL. Figure 2B shows resistance of the HLA modified clones to CTL-mediated lysis. Parental HEK293 and derived HLA-A^{neg} clones were cultured with IFN-γ and TNF-α for 48 hours and pulsed with serial dilutions of the cognate HLA-A3 peptide RVWDLPGVLK (SEQ ID NO:1, see also NP_001103685.1), derived from PANE1 (alternatively Centromere protein M isoform c) and recognized by CTL clone 7A7) or the HLA-A2 peptide CIPPDSLLFPA (SEQ ID NO:2, also alternative open reading frame of NM_199250.1) derived from C19ORF48/A2 and recognized by CTL clone GAS2B3-5) and evaluated for recognition by CTL clones in a 4-hour ⁵¹Cr release assay at an effector to target ratio of 20:1.HLA-A2⁺ LCL (hatched bar) that expresses PANE1 mHAg (not peptide-loaded) were used as a positive control.

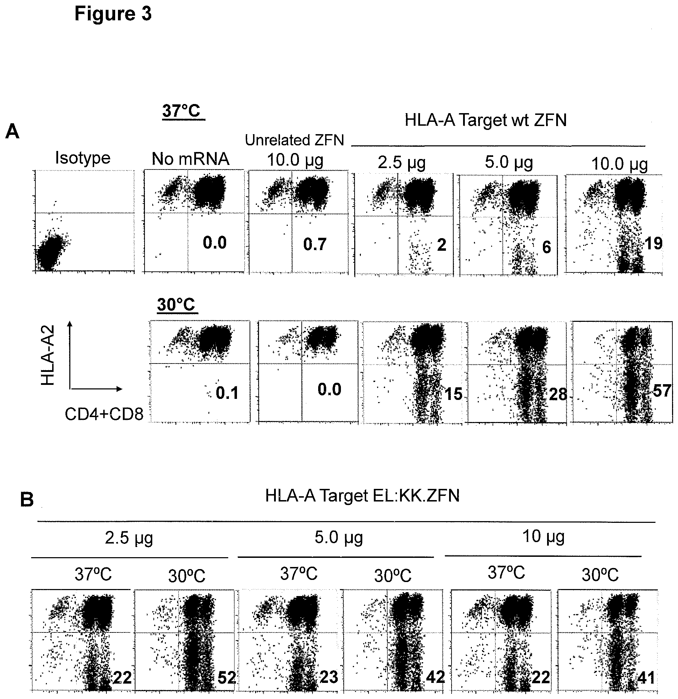

**Figure 3****, panels A and B,** show loss of HLA-A expression on primary OKT3-propagated T cells after genetic editing with ZFNs. Figure 3A (top panel) shows loss of cell surface expression of HLA-A2 after electro-transfer of mRNA species encoding ZFN-L and ZFN-R targeting HLA-A2 (SBS#18889 and SBS#18881, respectively, see U.S. Patent Publication No. 20120060230). Coexpression of HLA-A2, CD4, and CD8 were analyzed 4 days after electro-transfer of graded doses of the mRNA species encoding ZFN-L and ZFN-R. Flow cytometry data were gated on the propidium iodide-negative, live cell population. Numbers in the lower right quadrant indicate the percentage of CD4 and CD8+ T cells that are HLA-A^{neg}. Figure 3A (bottom panel) shows improved disruption of HLA-A expression by "cold shock." Data were collected 4 days after electro-transfer of graded doses of the mRNA species encoding ZFN-L and ZFN-R. Cells were cultured at 30°C from days 1 to 3 after electro-transfer of ZFNs, returned to 37°C and cultured for one additional day before analysis. Figure 3B shows improved efficiency of HLA-A disruption by ZFN-L and ZFN-R fused to the heterodimeric Fok I domain variants. mRNA species encoding the ZFN-L and ZFN-R heterodimeric Fok I mutants EL:KK targeting HLA-A were electro-transferred into primary T cells. HLA-A2 expression was analyzed after culturing the cells for 4 days at 37°C or 3 days at 30°C followed by 37°C for 1 day. X-axis represents CD4 and CD8 expression and y-axis represents HLA-A2 expression.

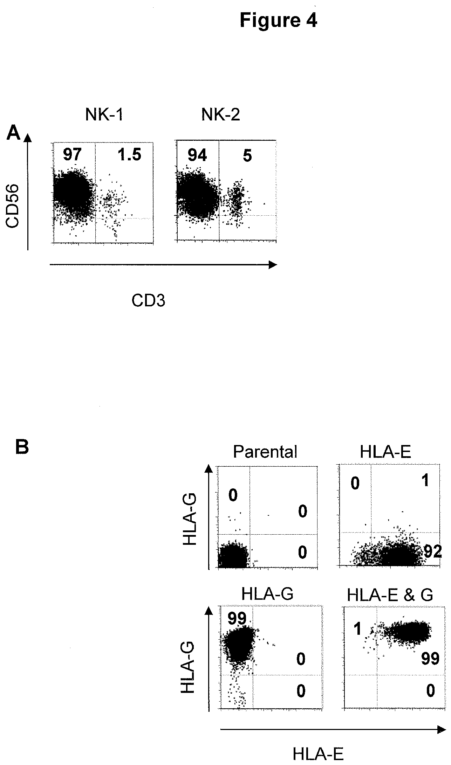

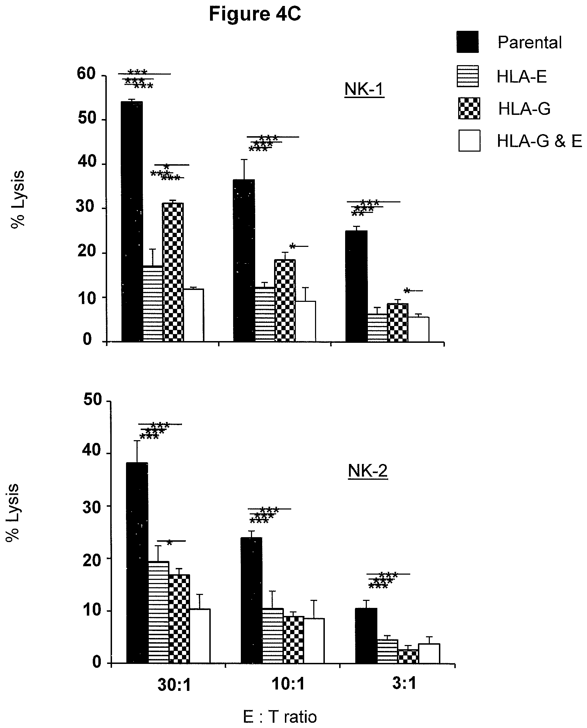

**Figure 4****, panels A to C,** show that expression of non-classical HLA molecules protects against NK-mediated cell lysis. Figure 4A shows the immunophenotype of NK cells isolated from two individual PBMCs from healthy donor (each donor designated as NK-1 and NK-2). Flow cytometry data shown are gated for PI^{neg} population. The numbers represent percentage of each upper quadrant. Figure 4B shows genetic modification of HLA class I^{low}721.221 cells to express HLA-E and/or HLA-G. The SB transposon/transposase system was used to homogenously express HLA-E and/or HLA-G in three clones of 721.221 cells. Each number represents percentage expression of HLA-G, HLA-E, or both HLA-G and HLA-E as detected by flow cytometry. Figure 4C shows specific lysis by NK cells targeting 721.221 cells. The relative ability of NK cells to kill parental (HLA class I^{low}), HLA-E⁺, HLA-G⁺, and both HLA-E⁺HLA-G⁺ 721.221 cells. Each column represents the mean ± standard deviation (SD) * .01< P < 0.05, **P < .01; and ***P < .001

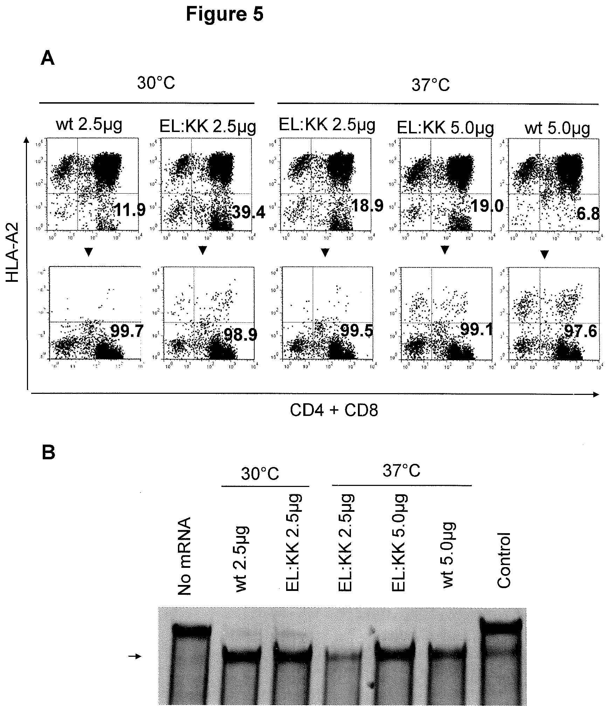

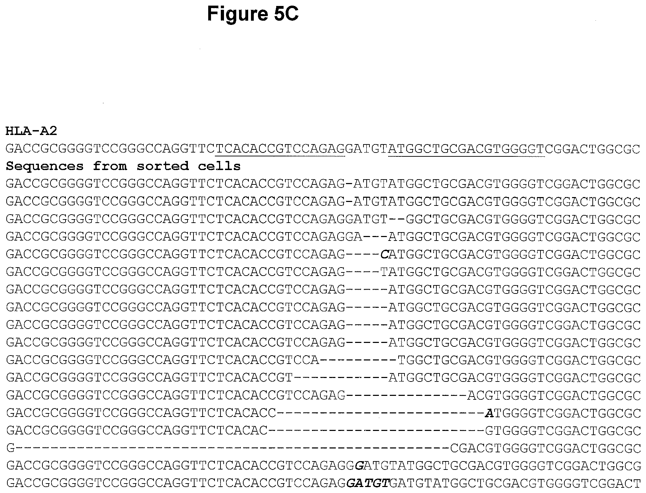

**Figure 5****, panels A to C,** shows enrichment of HLA-A^{neg} primary T cells after genetic editing with ZFNs. Figure 5A shows generation of an HLA-A2^{neg} T-cell population. HLA-A2^{neg} T cells were enriched by magnetic bead-based selection. Input dose of mRNA coding for ZFN and 3-day culture conditions (37°C versus 30°C) after electro-transfer of mRNA are indicated. The numbers represent HLA-A2 negative population within CD4 and CD8 positive population. Figure 5B shows Surveyor™ nuclease assay of the HLA-A2^{neg} T cells. Analysis of T cells enriched for loss of HLA-A2 expression demonstrates disruption in the HLA-A2 locus by the appearance of fast-moving band (arrow). Figure 5C shows results of sequencing of the HLA^{neg} T cells (SEQ ID NOs:39 to 53). PCR products using HLA-A2-specific primers from enriched cell (2.5 µg ZFNs, EL:KK *Fok* I domain, 30°C treatment) were cloned into a TOPO vector (Invitrogen) and plasmid products were sequenced. The wild type sequence is listed at the top with the expected ZFN binding sites underlined. Shown below are the sequences obtained from the ZFN-treated and enriched cells. Deletions are indicated by hyphens and sequence changes are highlighted in bold. All 18 sequence changes result in frame shifts predicted to prevent protein translation.

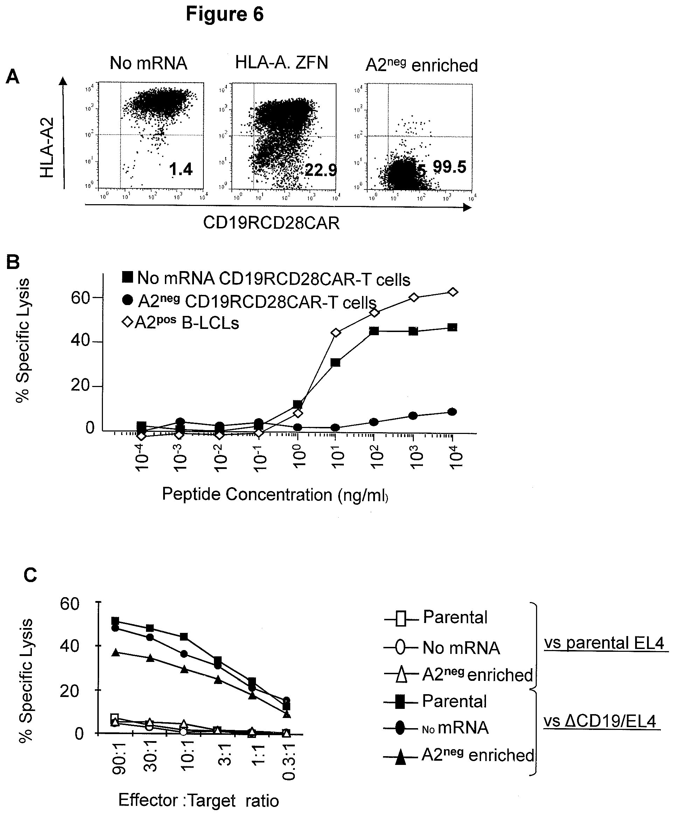

**Figure 6****, panels A to C,** show loss of HLA-A expression on primary CD19-specific CAR+ T cells genetically edited with ZFNs. Figure 6A shows disruption of HLA-A2 in CAR+ T cells by electro-transfer of mRNA encoding ZFNs. T cells from a HLA-A2+ donor were electroporated and propagated to express CD19-specific CAR (CD19RCD28). These T cells were re-electroporated with 2.5 µg of each mRNA encoding the heterodimeric *Fok* I domain variants of the HLA-A-specific ZFNs (ZFN-L-EL and ZFN-R-KK). HLA-A2 expression was analyzed after culturing at 30°C for 3 days followed by 37°C for 1 day. Enrichment of the HLA-A2^{neg} population was performed by paramagnetic selection. Figure 6B shows HLA-A^{neg} CAR+ T cells evade lysis by HLA-A2 restricted CTL. Pools of the indicated CAR+ T cells were pulsed with serial dilutions of cognate peptide before being used as targets in a CRA. CTL clone GAS2B3-5, which is specific for C19ORF48/A2, was added at an effector-to-target ratio of 20:1. Figure 6C shows ZFN-modified HLA^{neg} CAR+ T cells maintain desired antigen-specific cytotoxicity. Redirected specificity for CD19 by HLA-A^{neg}T cells expressing CD19RCD28 CAR was demonstrated using the mouse T-cell line EL4 genetically modified to expresses a truncated variant of human CD 19. Expression of introduced human CD 19 on EL4 was 100%.

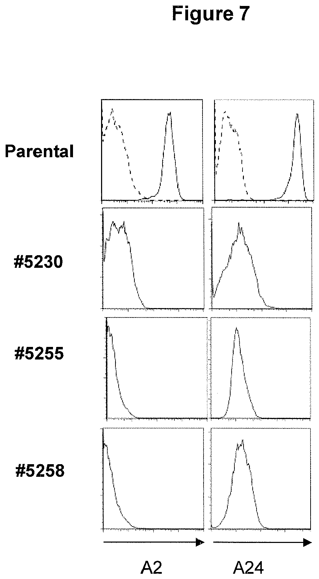

**Figure 7** shows ZFN-mediated elimination of HLA-A expression on human ESC. The HLAA2+HLA-24+hES parental cell line WIBR3 was modified by ZFN and donor plasmid coding for antibiotic resistance. Clones (5230, 5255, 5258) were chosen with loss of HLA-A expression and differentiated into fibroblasts. Expression of HLA-A2 and HLA-A24 on derived fibroblasts was assessed by flow cytometry after culturing with 600 IU/mL of IFN-γ and 10 ng/mL of TNF-α for 48 hours. Dashed line in parental panel represents isotype control.

### DETAILED DESCRIPTION

Disclosed herein are compositions and methods for generating cells in which one or more classic HLA genes are inactivated but which express one or more non-classic HLA genes. Cells modified targeted in this manner can be used as therapeutics, for example, transplants, as the presence of the non-classic HLA gene(s) reduces or eliminates NK-mediated lysis of HLA null cells. Additionally, other genes of interest may be inserted into cells in which the HLA genes have been manipulated.

Thus, the methods and compositions described herein provide methods for treatment of HLA related disorders, and these methods and compositions can comprise zinc finger transcription factors capable of modulating target genes as well as engineered zinc finger nucleases.

### General

Practice of the methods, as well as preparation and use of the compositions disclosed herein employ, unless otherwise indicated, conventional techniques in molecular biology, biochemistry, chromatin structure and analysis, computational chemistry, cell culture, recombinant DNA and related fields as are within the skill of the art. These techniques are fully explained in the literature. *See,* for example, Sambrook et al. MOLECULAR CLONING: A LABORATORY MANUAL, Second edition, Cold Spring Harbor Laboratory Press, 1989 and Third edition, 2001; Ausubel et al., CURRENT PROTOCOLS IN MOLECULAR BIOLOGY, John Wiley & Sons, New York, 1987 and periodic updates; the series METHODS IN ENZYMOLOGY, Academic Press, San Diego; Wolffe, CHROMATIN STRUCTURE AND FUNCTION, Third edition, Academic Press, San Diego, 1998; METHODS IN ENZYMOLOGY, Vol. 304, "Chromatin" (P.M. Wassarman and A. P. Wolffe, eds.), Academic Press, San Diego, 1999; and METHODS IN MOLECULAR BIOLOGY, Vol. 119, "Chromatin Protocols" (P.B. Becker, ed.) Humana Press, Totowa, 1999.

### Definitions

The terms "nucleic acid," "polynucleotide," and "oligonucleotide" are used interchangeably and refer to a deoxyribonucleotide or ribonucleotide polymer, in linear or circular conformation, and in either single- or double-stranded form. For the purposes of the present disclosure, these terms are not to be construed as limiting with respect to the length of a polymer. The terms can encompass known analogues of natural nucleotides, as well as nucleotides that are modified in the base, sugar and/or phosphate moieties (*e.g*., phosphorothioate backbones). In general, an analogue of a particular nucleotide has the same base-pairing specificity; *i.e.,* an analogue of A will base-pair with T.

The terms "polypeptide," "peptide" and "protein" are used interchangeably to refer to a polymer of amino acid residues. The term also applies to amino acid polymers in which one or more amino acids are chemical analogues or modified derivatives of corresponding naturally-occurring amino acids.

"Binding" refers to a sequence-specific, non-covalent interaction between macromolecules (*e.g*., between a protein and a nucleic acid). Not all components of a binding interaction need be sequence-specific (*e.g*., contacts with phosphate residues in a DNA backbone), as long as the interaction as a whole is sequence-specific. Such interactions are generally characterized by a dissociation constant (K_{d}) of 10⁻⁶ M⁻¹ or lower. "Affinity" refers to the strength of binding: increased binding affinity being correlated with a lower K_{d}.

A "binding protein" is a protein that is able to bind non-covalently to another molecule. A binding protein can bind to, for example, a DNA molecule (a DNA-binding protein), an RNA molecule (an RNA-binding protein) and/or a protein molecule (a protein-binding protein). In the case of a protein-binding protein, it can bind to itself (to form homodimers, homotrimers, *etc*.) and/or it can bind to one or more molecules of a different protein or proteins. A binding protein can have more than one type of binding activity. For example, zinc finger proteins have DNA-binding, RNA-binding and protein-binding activity.

A "zinc finger DNA binding protein" (or binding domain) is a protein, or a domain within a larger protein, that binds DNA in a sequence-specific manner through one or more zinc fingers, which are regions of amino acid sequence within the binding domain whose structure is stabilized through coordination of a zinc ion. The term zinc finger DNA binding protein is often abbreviated as zinc finger protein or ZFP.

A "TALE DNA binding domain" or "TALE" is a polypeptide comprising one or more TALE repeat domains/units. The repeat domains are involved in binding of the TALE to its cognate target DNA sequence. A single "repeat unit" (also referred to as a "repeat") is typically 33-35 amino acids in length and exhibits at least some sequence homology with other TALE repeat sequences within a naturally occurring TALE protein. *See, e.g.,* U.S. Patent No. 8,586,526.

Zinc finger and TALE DNA-binding domains can be "engineered" to bind to a predetermined nucleotide sequence, for example via engineering (altering one or more amino acids) of the recognition helix region of a naturally occurring zinc finger protein or by engineering of the amino acids involved in DNA binding (the repeat variable diresidue or RVD region). Therefore, engineered zinc finger proteins or TALE proteins are proteins that are non-naturally occurring. Non-limiting examples of methods for engineering zinc finger proteins and TALEs are design and selection. A designed protein is a protein not occurring in nature whose design/composition results principally from rational criteria. Rational criteria for design include application of substitution rules and computerized algorithms for processing information in a database storing information of existing ZFP or TALE designs and binding data. See, for example, U.S. Patent Nos. 8,586,526; 6,140,081; 6,453,242; and 6,534,261; see also WO 98/53058; WO 98/53059; WO 98/53060; WO 02/016536 and WO 03/016496.

A "selected" zinc finger protein or TALE is a protein not found in nature whose production results primarily from an empirical process such as phage display, interaction trap or hybrid selection. See *e.g.,* US 5,789,538; US 5,925,523; US 6,007,988; US 6,013,453; US 6,200,759; WO 95/19431; WO 96/06166; WO 98/53057; WO 98/54311; WO 00/27878; WO 01/60970 WO 01/88197 and WO 02/099084.

"Recombination" refers to a process of exchange of genetic information between two polynucleotides. For the purposes of this disclosure, "homologous recombination (HR)" refers to the specialized form of such exchange that takes place, for example, during repair of double-strand breaks in cells via homology-directed repair mechanisms. This process requires nucleotide sequence homology, uses a "donor" molecule to template repair of a "target" molecule (*i.e*., the one that experienced the double-strand break), and is variously known as "non-crossover gene conversion" or "short tract gene conversion," because it leads to the transfer of genetic information from the donor to the target. Without wishing to be bound by any particular theory, such transfer can involve mismatch correction of heteroduplex DNA that forms between the broken target and the donor, and/or "synthesis-dependent strand annealing," in which the donor is used to resynthesize genetic information that will become part of the target, and/or related processes. Such specialized HR often results in an alteration of the sequence of the target molecule such that part or all of the sequence of the donor polynucleotide is incorporated into the target polynucleotide.

In the methods of the disclosure, one or more targeted nucleases as described herein create a double-stranded break in the target sequence (*e.g*., cellular chromatin) at a predetermined site, and a "donor" polynucleotide, having homology to the nucleotide sequence in the region of the break, can be introduced into the cell. The presence of the double-stranded break has been shown to facilitate integration of the donor sequence. The donor sequence may be physically integrated or, alternatively, the donor polynucleotide is used as a template for repair of the break via homologous recombination, resulting in the introduction of all or part of the nucleotide sequence as in the donor into the cellular chromatin. Thus, a first sequence in cellular chromatin can be altered and, in certain embodiments, can be converted into a sequence present in a donor polynucleotide. Thus, the use of the terms "replace" or "replacement" can be understood to represent replacement of one nucleotide sequence by another, (*i.e.,* replacement of a sequence in the informational sense), and does not necessarily require physical or chemical replacement of one polynucleotide by another.

In any of the methods described herein, additional pairs of zinc-finger proteins can be used for additional double-stranded cleavage of additional target sites within the cell.

In certain embodiments of methods for targeted recombination and/or replacement and/or alteration of a sequence in a region of interest in cellular chromatin, a chromosomal sequence is altered by homologous recombination with an exogenous "donor" nucleotide sequence. Such homologous recombination is stimulated by the presence of a double-stranded break in cellular chromatin, if sequences homologous to the region of the break are present.

In any of the methods described herein, the first nucleotide sequence (the "donor sequence") can contain sequences that are homologous, but not identical, to genomic sequences in the region of interest, thereby stimulating homologous recombination to insert a non-identical sequence in the region of interest. Thus, in certain embodiments, portions of the donor sequence that are homologous to sequences in the region of interest exhibit between about 80 to 99% (or any integer therebetween) sequence identity to the genomic sequence that is replaced. In other embodiments, the homology between the donor and genomic sequence is higher than 99%, for example if only 1 nucleotide differs as between donor and genomic sequences of over 100 contiguous base pairs. In certain cases, a non-homologous portion of the donor sequence can contain sequences not present in the region of interest, such that new sequences are introduced into the region of interest. In these instances, the non-homologous sequence is generally flanked by sequences of 50-1,000 base pairs (or any integral value therebetween) or any number of base pairs greater than 1,000, that are homologous or identical to sequences in the region of interest. In other embodiments, the donor sequence is non-homologous to the first sequence, and is inserted into the genome by non-homologous recombination mechanisms.

Any of the methods described herein can be used for partial or complete inactivation of one or more target sequences in a cell by targeted integration of donor sequence that disrupts expression of the gene(s) of interest. Cell lines with partially or completely inactivated genes are also provided.

Furthermore, the methods of targeted integration as described herein can also be used to integrate one or more exogenous sequences. The exogenous nucleic acid sequence can comprise, for example, one or more genes or cDNA molecules, or any type of coding or noncoding sequence, as well as one or more control elements (*e.g*., promoters). In addition, the exogenous nucleic acid sequence may produce one or more RNA molecules (*e.g*., small hairpin RNAs (shRNAs), inhibitory RNAs (RNAis), microRNAs (miRNAs), *etc*.)*.*

"Cleavage" refers to the breakage of the covalent backbone of a DNA molecule. Cleavage can be initiated by a variety of methods including, but not limited to, enzymatic or chemical hydrolysis of a phosphodiester bond. Both single-stranded cleavage and double-stranded cleavage are possible, and double-stranded cleavage can occur as a result of two distinct single-stranded cleavage events. DNA cleavage can result in the production of either blunt ends or staggered ends. In certain embodiments, fusion polypeptides are used for targeted double-stranded DNA cleavage.

A "cleavage half-domain" is a polypeptide sequence which, in conjunction with a second polypeptide (either identical or different) forms a complex having cleavage activity (preferably double-strand cleavage activity). The terms "first and second cleavage half-domains;" "+ and - cleavage half-domains" and "right and left cleavage half-domains" are used interchangeably to refer to pairs of cleavage half-domains that dimerize.

An "engineered cleavage half-domain" is a cleavage half-domain that has been modified so as to form obligate heterodimers with another cleavage half-domain (*e.g.,* another engineered cleavage half-domain). *See, also,* U.S. Patent Nos. 7,888,121; 7,914,796; 8,034,598; 8,623,618 and U.S. Patent Publication No. 2011/0201055.

The term "sequence" refers to a nucleotide sequence of any length, which can be DNA or RNA; can be linear, circular or branched and can be either single-stranded or double stranded. The term "donor sequence" refers to a nucleotide sequence that is inserted into a genome. A donor sequence can be of any length, for example between 2 and 10,000 nucleotides in length (or any integer value therebetween or thereabove), preferably between about 100 and 1,000 nucleotides in length (or any integer therebetween), more preferably between about 200 and 500 nucleotides in length.

"Chromatin" is the nucleoprotein structure comprising the cellular genome. Cellular chromatin comprises nucleic acid, primarily DNA, and protein, including histones and non-histone chromosomal proteins. The majority of eukaryotic cellular chromatin exists in the form of nucleosomes, wherein a nucleosome core comprises approximately 150 base pairs of DNA associated with an octamer comprising two each of histones H2A, H2B, H3 and H4; and linker DNA (of variable length depending on the organism) extends between nucleosome cores. A molecule of histone HI is generally associated with the linker DNA. For the purposes of the present disclosure, the term "chromatin" is meant to encompass all types of cellular nucleoprotein, both prokaryotic and eukaryotic. Cellular chromatin includes both chromosomal and episomal chromatin.

A "chromosome," is a chromatin complex comprising all or a portion of the genome of a cell. The genome of a cell is often characterized by its karyotype, which is the collection of all the chromosomes that comprise the genome of the cell. The genome of a cell can comprise one or more chromosomes.

An "episome" is a replicating nucleic acid, nucleoprotein complex or other structure comprising a nucleic acid that is not part of the chromosomal karyotype of a cell. Examples of episomes include plasmids and certain viral genomes.

A "target site" or "target sequence" is a nucleic acid sequence that defines a portion of a nucleic acid to which a binding molecule will bind, provided sufficient conditions for binding exist. For example, the sequence 5' GAATTC 3' is a target site for the Eco RI restriction endonuclease.

An "exogenous" molecule is a molecule that is not normally present in a cell, but can be introduced into a cell by one or more genetic, biochemical or other methods. "Normal presence in the cell" is determined with respect to the particular developmental stage and environmental conditions of the cell. Thus, for example, a molecule that is present only during embryonic development of muscle is an exogenous molecule with respect to an adult muscle cell. Similarly, a molecule induced by heat shock is an exogenous molecule with respect to a non-heat-shocked cell. An exogenous molecule can comprise, for example, a functioning version of a malfunctioning endogenous molecule or a malfunctioning version of a normally-functioning endogenous molecule.

An exogenous molecule can be, among other things, a small molecule, such as is generated by a combinatorial chemistry process, or a macromolecule such as a protein, nucleic acid, carbohydrate, lipid, glycoprotein, lipoprotein, polysaccharide, any modified derivative of the above molecules, or any complex comprising one or more of the above molecules. Nucleic acids include DNA and RNA, can be single- or double-stranded; can be linear, branched or circular; and can be of any length. Nucleic acids include those capable of forming duplexes, as well as triplex-forming nucleic acids. See, for example, U.S. Patent Nos. 5,176,996 and 5,422,251. Proteins include, but are not limited to, DNA-binding proteins, transcription factors, chromatin remodeling factors, methylated DNA binding proteins, polymerases, methylases, demethylases, acetylases, deacetylases, kinases, phosphatases, integrases, recombinases, ligases, topoisomerases, gyrases and helicases.

An exogenous molecule can be the same type of molecule as an endogenous molecule, *e.g*., an exogenous protein or nucleic acid. For example, an exogenous nucleic acid can comprise an infecting viral genome, a plasmid or episome introduced into a cell, or a chromosome that is not normally present in the cell. Methods for the introduction of exogenous molecules into cells are known to those of skill in the art and include, but are not limited to, lipid-mediated transfer (*i.e.,* liposomes, including neutral and cationic lipids), electroporation, direct injection, cell fusion, particle bombardment, calcium phosphate co-precipitation, DEAE-dextranmediated transfer and viral vector-mediated transfer. An exogenous molecule can also be the same type of molecule as an endogenous molecule but derived from a different species than the cell is derived from. For example, a human nucleic acid sequence may be introduced into a cell line originally derived from a mouse or hamster.

By contrast, an "endogenous" molecule is one that is normally present in a particular cell at a particular developmental stage under particular environmental conditions. For example, an endogenous nucleic acid can comprise a chromosome, the genome of a mitochondrion, chloroplast or other organelle, or a naturally-occurring episomal nucleic acid. Additional endogenous molecules can include proteins, for example, transcription factors and enzymes.

A "fusion" molecule is a molecule in which two or more subunit molecules are linked, preferably covalently. The subunit molecules can be the same chemical type of molecule, or can be different chemical types of molecules. Examples of the first type of fusion molecule include, but are not limited to, fusion proteins (for example, a fusion between a ZFP or TALE DNA-binding domain and one or more activation domains) and fusion nucleic acids (for example, a nucleic acid encoding the fusion protein described *supra*). Examples of the second type of fusion molecule include, but are not limited to, a fusion between a triplex-forming nucleic acid and a polypeptide, and a fusion between a minor groove binder and a nucleic acid.

Expression of a fusion protein in a cell can result from delivery of the fusion protein to the cell or by delivery of a polynucleotide encoding the fusion protein to a cell, wherein the polynucleotide is transcribed, and the transcript is translated, to generate the fusion protein. Trans-splicing, polypeptide cleavage and polypeptide ligation can also be involved in expression of a protein in a cell. Methods for polynucleotide and polypeptide delivery to cells are presented elsewhere in this disclosure.

A "gene," for the purposes of the present disclosure, includes a DNA region encoding a gene product (see *infra*), as well as all DNA regions which regulate the production of the gene product, whether or not such regulatory sequences are adjacent to coding and/or transcribed sequences. Accordingly, a gene includes, but is not necessarily limited to, promoter sequences, terminators, translational regulatory sequences such as ribosome binding sites and internal ribosome entry sites, enhancers, silencers, insulators, boundary elements, replication origins, matrix attachment sites and locus control regions.

"Gene expression" refers to the conversion of the information, contained in a gene, into a gene product. A gene product can be the direct transcriptional product of a gene (*e.g*., mRNA, tRNA, rRNA, antisense RNA, ribozyme, structural RNA or any other type of RNA) or a protein produced by translation of an mRNA. Gene products also include RNAs which are modified, by processes such as capping, polyadenylation, methylation, and editing, and proteins modified by, for example, methylation, acetylation, phosphorylation, ubiquitination, ADP-ribosylation, myristilation, and glycosylation.

"Modulation" of gene expression refers to a change in the activity of a gene. Modulation of expression can include, but is not limited to, gene activation and gene repression. Genome editing (*e.g*., cleavage, alteration, inactivation, random mutation) can be used to modulate expression. Gene inactivation refers to any reduction in gene expression as compared to a cell that does not include a ZFP as described herein. Thus, gene inactivation may be partial or complete.

A "region of interest" is any region of cellular chromatin, such as, for example, a gene or a non-coding sequence within or adjacent to a gene, in which it is desirable to bind an exogenous molecule. Binding can be for the purposes of targeted DNA cleavage and/or targeted recombination. A region of interest can be present in a chromosome, an episome, an organellar genome (*e.g*., mitochondrial, chloroplast), or an infecting viral genome, for example. A region of interest can be within the coding region of a gene, within transcribed non-coding regions such as, for example, leader sequences, trailer sequences or introns, or within non-transcribed regions, either upstream or downstream of the coding region. A region of interest can be as small as a single nucleotide pair or up to 2,000 nucleotide pairs in length, or any integral value of nucleotide pairs.

"Eukaryotic" cells include, but are not limited to, fungal cells (such as yeast), plant cells, animal cells, mammalian cells and human cells (*e.g.*, T-cells).

The terms "operative linkage" and "operatively linked" (or "operably linked") are used interchangeably with reference to a juxtaposition of two or more components (such as sequence elements), in which the components are arranged such that both components function normally and allow the possibility that at least one of the components can mediate a function that is exerted upon at least one of the other components. By way of illustration, a transcriptional regulatory sequence, such as a promoter, is operatively linked to a coding sequence if the transcriptional regulatory sequence controls the level of transcription of the coding sequence in response to the presence or absence of one or more transcriptional regulatory factors. A transcriptional regulatory sequence is generally operatively linked in *cis* with a coding sequence, but need not be directly adjacent to it. For example, an enhancer is a transcriptional regulatory sequence that is operatively linked to a coding sequence, even though they are not contiguous.

With respect to fusion polypeptides, the term "operatively linked" can refer to the fact that each of the components performs the same function in linkage to the other component as it would if it were not so linked. For example, with respect to a fusion polypeptide in which a DNA-binding domain (*e.g*., ZFP, TALE) is fused to an activation domain, the DNA-binding domain and the activation domain are in operative linkage if, in the fusion polypeptide, the DNA-binding domain portion is able to bind its target site and/or its binding site, while the activation domain is able to up-regulate gene expression. When a fusion polypeptide in which a DNA-binding domain is fused to a cleavage domain, the DNA-binding domain and the cleavage domain are in operative linkage if, in the fusion polypeptide, the DNA-binding domain portion is able to bind its target site and/or its binding site, while the cleavage domain is able to cleave DNA in the vicinity of the target site. Similarly, with respect to a fusion polypeptide in which a DNA-binding domain is fused to an activation or repression domain, the DNA-binding domain and the activation or repression domain are in operative linkage if, in the fusion polypeptide, the DNA-binding domain portion is able to bind its target site and/or its binding site, while the activation domain is able to upregulate gene expression or the repression domain is able to downregulate gene expression.

A "functional fragment" of a protein, polypeptide or nucleic acid is a protein, polypeptide or nucleic acid whose sequence is not identical to the full-length protein, polypeptide or nucleic acid, yet retains the same function as the full-length protein, polypeptide or nucleic acid. A functional fragment can possess more, fewer, or the same number of residues as the corresponding native molecule, and/or can contain one or more amino acid or nucleotide substitutions. Methods for determining the function of a nucleic acid (*e.g*., coding function, ability to hybridize to another nucleic acid) are well-known in the art. Similarly, methods for determining protein function are well-known. For example, the DNA-binding function of a polypeptide can be determined, for example, by filter-binding, electrophoretic mobility-shift, or immunoprecipitation assays. DNA cleavage can be assayed by gel electrophoresis. See Ausubel *et al.*, *supra.* The ability of a protein to interact with another protein can be determined, for example, by co-immunoprecipitation, two-hybrid assays or complementation, both genetic and biochemical. See, for example, Fields et al. (1989) Nature 340:245-246; U.S. Patent No. 5,585,245 and PCT WO 98/44350.

A "vector" is capable of transferring gene sequences to target cells. Typically, "vector construct," "expression vector," and "gene transfer vector," mean any nucleic acid construct capable of directing the expression of a gene of interest and which can transfer gene sequences to target cells. Thus, the term includes cloning, and expression vehicles, as well as integrating vectors.

A "reporter gene" or "reporter sequence" refers to any sequence that produces a protein product that is easily measured, preferably although not necessarily in a routine assay. Suitable reporter genes include, but are not limited to, sequences encoding proteins that mediate antibiotic resistance (*e.g*., ampicillin resistance, neomycin resistance, G418 resistance, puromycin resistance), sequences encoding colored or fluorescent or luminescent proteins (*e.g*., green fluorescent protein, enhanced green fluorescent protein, red fluorescent protein, luciferase), and proteins which mediate enhanced cell growth and/or gene amplification (*e.g*., dihydrofolate reductase). Epitope tags include, for example, one or more copies of FLAG, His, myc, Tap, HA or any detectable amino acid sequence. "Expression tags" include sequences that encode reporters that may be operably linked to a desired gene sequence in order to monitor expression of the gene of interest.

### DNA-binding domains

Described herein are compositions comprising a DNA-binding domain that specifically binds to a target site in any gene comprising a HLA gene or a HLA regulator. Any DNA-binding domain can be used in the compositions and methods disclosed herein.

In certain embodiments, the DNA binding domain comprises a zinc finger protein. Preferably, the zinc finger protein is non-naturally occurring in that it is engineered to bind to a target site of choice. *See,* for example, Beerli et al. (2002) Nature Biotechnol. 20:135-141; Pabo et al. (2001) Ann. Rev. Biochem. 70:313-340; Isalan et al. (2001) Nature Biotechnol. 19:656-660; Segal et al. (2001) Curr. Opin. Biotechnol. 12:632-637; Choo et al. (2000) Curr. Opin. Struct. Biol. 10:411-416; U.S. Patent Nos. 6,453,242; 6,534,261; 6,599,692; 6,503,717; 6,689,558; 7,030,215; 6,794,136; 7,067,317; 7,262,054; 7,070,934; 7,361,635; 7,253,273; and U.S. Patent Publication Nos. 2005/0064474; 2007/0218528; 2005/0267061. In certain embodiments, the DNA-binding domain comprises a zinc finger protein disclosed in U.S. Patent Publication No. 2012/0060230 (*e.g.,* Table 1).

An engineered zinc finger binding domain can have a novel binding specificity, compared to a naturally-occurring zinc finger protein. Engineering methods include, but are not limited to, rational design and various types of selection. Rational design includes, for example, using databases comprising triplet (or quadruplet) nucleotide sequences and individual zinc finger amino acid sequences, in which each triplet or quadruplet nucleotide sequence is associated with one or more amino acid sequences of zinc fingers which bind the particular triplet or quadruplet sequence. See, for example, U.S. Patents 6,453,242 and 6,534,261.

Exemplary selection methods, including phage display and two-hybrid systems, are disclosed in US Patents 5,789,538; 5,925,523; 6,007,988; 6,013,453; 6,410,248; 6,140,466; 6,200,759; and 6,242,568; as well as WO 98/37186; WO 98/53057; WO 00/27878; WO 01/88197 and GB 2,338,237. In addition, enhancement of binding specificity for zinc finger binding domains has been described, for example, in U.S. Patent No. 6,794,136.

In addition, as disclosed in these and other references, zinc finger domains and/or multi-fingered zinc finger proteins may be linked together using any suitable linker sequences, including for example, linkers of 5 or more amino acids in length. See, also, U.S. Patent Nos. 6,479,626; 6,903,185; and 7,153,949 for exemplary linker sequences 6 or more amino acids in length. The proteins described herein may include any combination of suitable linkers between the individual zinc fingers of the protein. In addition, enhancement of binding specificity for zinc finger binding domains has been described, for example, in U.S. Patent No. 6,794,136.

Selection of target sites; ZFPs and methods for design and construction of fusion proteins (and polynucleotides encoding same) are known to those of skill in the art and described in detail in U.S. Patent Nos. 6,140,0815; 789,538; 6,453,242; 6,534,261; 5,925,523; 6,007,988; 6,013,453; 6,200,759; WO 95/19431; WO 96/06166; WO 98/53057; WO 98/54311; WO 00/27878; WO 01/60970 WO 01/88197; WO 02/099084; WO 98/53058; WO 98/53059; WO 98/53060; WO 02/016536 and WO 03/016496.

In addition, as disclosed in these and other references, zinc finger domains and/or multi-fingered zinc finger proteins may be linked together using any suitable linker sequences, including for example, linkers of 5 or more amino acids in length. See, also, U.S. Patent Nos. 6,479,626; 6,903,185; and 7,153,949 for exemplary linker sequences 6 or more amino acids in length. The proteins described herein may include any combination of suitable linkers between the individual zinc fingers of the protein.

In certain embodiments, the DNA binding domain is an engineered zinc finger protein that binds (in a sequence-specific manner) to a target site in a HLA gene or HLA regulatory gene and modulates expression of HLA. The ZFPs can bind selectively to a specific haplotype of interest. For a discussion of HLA haplotypes identified in the United States population and their frequency according to different races, see Maiers et al (2007) Human Immunology 68: 779- 788.

Additionally, ZFPs are provided that bind to functional HLA regulator genes including, but not limited to, Tap1, Tap2, Tapascin, CTFIIA, and RFX5. HLA target sites typically include at least one zinc finger but can include a plurality of zinc fingers (*e.g.*, 2, 3, 4, 5, 6 or more fingers). Usually, the ZFPs include at least three fingers. Certain of the ZFPs include four, five or six fingers. The ZFPs that include three fingers typically recognize a target site that includes 9 or 10 nucleotides; ZFPs that include four fingers typically recognize a target site that includes 12 to 14 nucleotides; while ZFPs having six fingers can recognize target sites that include 18 to 21 nucleotides. The ZFPs can also be fusion proteins that include one or more regulatory domains, which domains can be transcriptional activation or repression domains.

Specific examples of ZFPs are disclosed in Table 1 of U.S. Patent Publication No. 20120060230.

In some embodiments, the DNA-binding domain may be derived from a nuclease. For example, the recognition sequences of homing endonucleases and meganucleases such as I*-Sce*I*,* I*-Ceu*I*,* PI*-Psp*I*,* PI*-Sce,* I*-Sce*IV*,* I*-Csm*I*,* I*-Pan*I*, I-Sce*II*,* I*-Ppo*I*,* I-*Sce*III, I*-Cre*I*,* I*-Tev*I*,* I*-Tev*II and I-T*ev*III are known. *See* also U.S. Patent No. 5,420,032; U.S. Patent No. 6,833,252; Belfort et al. (1997) Nucleic Acids Res. 25:3379-3388; Dujon et al. (1989) Gene 82:115-118; Perler et al. (1994) Nucleic Acids Res. 22, 1125-1127; Jasin (1996) Trends Genet. 12:224-228; Gimble etal. (1996) J. Mol. Biol. 263:163-180; Argast et al. (1998) J. Mol. Biol. 280:345-353 and the New England Biolabs catalogue. In addition, the DNA-binding specificity of homing endonucleases and meganucleases can be engineered to bind non-natural target sites. *See,* for example, Chevalier et al. (2002) Molec. Cell 10:895-905; Epinat et al. (2003) Nucleic Acids Res. 31:2952-2962; Ashworth et al. (2006) Nature 441:656-659; Paques et al. (2007) Current Gene Therapy 7:49-66; U.S. Patent Publication No. 20070117128.

In other embodiments, the DNA binding domain comprises an engineered domain from a TAL effector similar to those derived from the plant pathogens *Xanthomonas* (see Boch et al, (2009) Science 326: 1509-1512 and Moscou and Bogdanove, (2009) Science326: 1501) and Ralstonia (see Heuer et al (2007) Applied and Environmental Microbiology 73(13): 4379-4384); U.S. Patent Application Nos. 20110301073 and 20110145940. The plant pathogenic bacteria of the genus *Xanthomonas* are known to cause many diseases in important crop plants. Pathogenicity of *Xanthomonas* depends on a conserved type III secretion (T3S) system which injects more than 25 different effector proteins into the plant cell. Among these injected proteins are transcription activator-like effectors (TALE) which mimic plant transcriptional activators and manipulate the plant transcriptome (see Kay et al (2007) Science318:648-651). These proteins contain a DNA binding domain and a transcriptional activation domain. One of the most well characterized TALEs is AvrBs3 from *Xanthomonas campestgris* pv. *Vesicatoria* (see Bonas et al (1989) Mol Gen Genet 218: 127-136 and WO2010079430). TALEs contain a centralized domain of tandem repeats, each repeat containing approximately 34 amino acids, which are key to the DNA binding specificity of these proteins. In addition, they contain a nuclear localization sequence and an acidic transcriptional activation domain (for a review see Schornack S, et al (2006) J Plant Physiol 163(3): 256-272). In addition, in the phytopathogenic bacteria *Ralstonia solanacearum* two genes, designated brg11 and hpx17 have been found that are homologous to the AvrBs3 family of *Xanthomonas* in the *R. solanacearum* biovar 1 strain GMI1000 and in the biovar 4 strain RS1000 (*See* Heuer et al (2007) Appl and Envir Micro 73(13): 4379-4384). These genes are 98.9% identical in nucleotide sequence to each other but differ by a deletion of 1,575 bp in the repeat domain of hpx17. However, both gene products have less than 40% sequence identity with AvrBs3 family proteins of *Xanthomonas.*

In addition, as disclosed in these and other references, zinc finger domains and/or multi-fingered zinc finger proteins or TALEs may be linked together using any suitable linker sequences, including for example, linkers of 5 or more amino acids in length. See, also, U.S. Patent Nos. 6,479,626; 6,903,185; and 7,153,949 for exemplary linker sequences 6 or more amino acids in length. The proteins described herein may include any combination of suitable linkers between the individual zinc fingers of the protein. In addition, enhancement of binding specificity for zinc finger binding domains has been described, for example, in U.S. Patent No. 6,794,136.

### Fusion proteins

Fusion proteins comprising DNA-binding proteins (*e.g*., ZFPs or TALEs) as described herein and a heterologous regulatory (functional) domain (or functional fragment thereof) are also provided. Common domains include, *e.g*., transcription factor domains (activators, repressors, co-activators, co-repressors), silencers, oncogenes (*e.g*., myc, jun, fos, myb, max, mad, rel, ets, bcl, myb, mos family members etc.); DNA repair enzymes and their associated factors and modifiers; DNA rearrangement enzymes and their associated factors and modifiers; chromatin associated proteins and their modifiers (*e.g*. kinases, acetylases and deacetylases); and DNA modifying enzymes (*e.g*., methyltransferases, topoisomerases, helicases, ligases, kinases, phosphatases, polymerases, endonucleases) and their associated factors and modifiers. U.S. Patent Application Publication Nos. 20050064474; 20060188987 and 2007/0218528 for details regarding fusions of DNA-binding domains and nuclease cleavage domains.

Suitable domains for achieving activation include the HSV VP16 activation domain (*see, e.g.,* Hagmann et al., J. Virol. 71, 5952-5962 (1997)) nuclear hormone receptors (*see, e.g.,* Torchia et al., Curr. Opin. Cell. Biol. 10:373-383 (1998)); the p65 subunit of nuclear factor kappa B (Bitko & Barik, J. Virol. 72:5610-5618 (1998) and Doyle & Hunt, Neuroreport 8:2937-2942 (1997)); Liu et al., Cancer Gene Ther. 5:3-28 (1998)), or artificial chimeric functional domains such as VP64 (Beerli et al., (1998) Proc. Natl. Acad. Sci. USA 95:14623-33), and degron (Molinari et al., (1999) EMBO J. 18, 6439-6447). Additional exemplary activation domains include, Oct 1, Oct-2A, Sp1, AP-2, and CTF1 (Seipel et al., EMBO J. 11, 4961-4968 (1992) as well as p300, CBP, PCAF, SRC1 PvALF, AtHD2A and ERF-2. *See,* for example, Robyr et al. (2000) Mol. Endocrinol. 14:329-347; Collingwood et al. (1999) J. Mol. Endocrinol. 23:255-275; Leo et al. (2000) Gene 245:1-11; Manteuffel-Cymborowska (1999) Acta Biochim. Pol. 46:77-89; McKenna et al. (1999) J. Steroid Biochem. Mol. Biol. 69:3-12; Malik et al. (2000) Trends Biochem. Sci. 25:277-283; and Lemon et al. (1999) Curr. Opin. Genet. Dev. 9:499-504. Additional exemplary activation domains include, but are not limited to, OsGAI, HALF-1, C1, API, ARF-5,-6,-7, and -8, CPRF1, CPRF4, MYC-RP/GP, and TRAB1. *See,* for example, Ogawa et al. (2000) Gene 245:21-29; Okanami et al. (1996) Genes Cells 1:87-99; Goff et al. (1991) Genes Dev. 5:298-309; Cho et al. (1999) Plant Mol. Biol. 40:419-429; Ulmason et al. (1999) Proc. Natl. Acad. Sci. USA 96:5844-5849; Sprenger-Haussels et al. (2000) Plant J. 22:1-8; Gong et al. (1999) Plant Mol. Biol. 41:33-44; and Hobo et al. (1999) Proc. Natl. Acad. Sci. USA 96:15,348-15,353.

It will be clear to those of skill in the art that, in the formation of a fusion protein (or a nucleic acid encoding same) between a DNA-binding domain and a functional domain, either an activation domain or a molecule that interacts with an activation domain is suitable as a functional domain. Essentially any molecule capable of recruiting an activating complex and/or activating activity (such as, for example, histone acetylation) to the target gene is useful as an activating domain of a fusion protein. Insulator domains, localization domains, and chromatin remodeling proteins such as ISWI-containing domains and/or methyl binding domain proteins suitable for use as functional domains in fusion molecules are described, for example, in U.S. Patent Applications 2002/0115215 and 2003/0082552 and in WO 02/44376.

Exemplary repression domains include, but are not limited to, KRAB A/B, KOX, TGF-beta-inducible early gene (TIEG), v-erbA, SID, MBD2, MBD3, members of the DNMT family (*e.g*., DNMT1, DNMT3A, DNMT3B), Rb, and MeCP2. *See,* for example, Bird et al. (1999) Cell 99:451-454; Tyler et al. (1999) Cell 99:443-446; Knoepfler et al. (1999) Cell 99:447-450; and Robertson et al. (2000) Nature Genet. 25:338-342. Additional exemplary repression domains include, but are not limited to, ROM2 and AtHD2A. *See,* for example, Chem et al. (1996) Plant Cell 8:305-321; and Wu et al. (2000) Plant J. 22:19-27.

Fusion molecules are constructed by methods of cloning and biochemical conjugation that are well known to those of skill in the art. Fusion molecules comprise a DNA-binding domain and a functional domain (*e.g*., a transcriptional activation or repression domain). Fusion molecules also optionally comprise nuclear localization signals (such as, for example, that from the SV40 medium T-antigen) and epitope tags (such as, for example, FLAG and hemagglutinin). Fusion proteins (and nucleic acids encoding them) are designed such that the translational reading frame is preserved among the components of the fusion.

Fusions between a polypeptide component of a functional domain (or a functional fragment thereof) on the one hand, and a non-protein DNA-binding domain (*e.g*., antibiotic, intercalator, minor groove binder, nucleic acid) on the other, are constructed by methods of biochemical conjugation known to those of skill in the art. *See,* for example, the Pierce Chemical Company (Rockford, IL) Catalogue. Methods and compositions for making fusions between a minor groove binder and a polypeptide have been described. Mapp et al. (2000) Proc. Natl. Acad. Sci. USA 97:3930-3935.

In certain embodiments, the target site bound by the zinc finger protein is present in an accessible region of cellular chromatin. Accessible regions can be determined as described, for example, in U.S. Patent Nos. 7,217,509 and 7,923,542. If the target site is not present in an accessible region of cellular chromatin, one or more accessible regions can be generated as described in U.S. Patent Nos. 7,785,792 and 8,071,370. In additional embodiments, the DNA-binding domain of a fusion molecule is capable of binding to cellular chromatin regardless of whether its target site is in an accessible region or not. For example, such DNA-binding domains are capable of binding to linker DNA and/or nucleosomal DNA. Examples of this type of "pioneer" DNA binding domain are found in certain steroid receptor and in hepatocyte nuclear factor 3 (HNF3). Cordingley et al. (1987) Cell 48:261-270; Pina et al. (1990) Cell 60:719-731; and Cirillo et al. (1998) EMBO J. 17:244-254.

The fusion molecule may be formulated with a pharmaceutically acceptable carrier, as is known to those of skill in the art. *See,* for example, Remington's Pharmaceutical Sciences, 17th ed., 1985; and U.S. Patent Nos. 6,453,242 and 6,534,261.

The functional component/domain of a fusion molecule can be selected from any of a variety of different components capable of influencing transcription of a gene once the fusion molecule binds to a target sequence via its DNA binding domain. Hence, the functional component can include, but is not limited to, various transcription factor domains, such as activators, repressors, co-activators, co-repressors, and silencers.

Additional exemplary functional domains are disclosed, for example, in U.S. Patent Nos. 6,534,261 and 6,933,113.

Functional domains that are regulated by exogenous small molecules or ligands may also be selected. For example, RheoSwitch® technology may be employed wherein a functional domain only assumes its active conformation in the presence of the external RheoChem™ ligand (*see* for example US 20090136465). Thus, the ZFP may be operably linked to the regulatable functional domain wherein the resultant activity of the ZFP-TF is controlled by the external ligand.

### Nucleases

In certain embodiments, the fusion protein comprises a DNA-binding binding domain and cleavage (nuclease) domain. As such, gene modification can be achieved using a nuclease, for example an engineered nuclease. Engineered nuclease technology is based on the engineering of naturally occurring DNA-binding proteins. For example, engineering of homing endonucleases with tailored DNA-binding specificities has been described. Chames et al. (2005) Nucleic Acids Res 33(20):e178; Arnould et al. (2006) J. Mol. Biol. 355:443-458. In addition, engineering of ZFPs has also been described. See, *e.g.,* U.S. Patent Nos. 6,534,261; 6,607,882; 6,824,978; 6,979,539; 6,933,113; 7,163,824; and 7,013,219.

In addition, ZFPs and/or TALEs have been fused to nuclease domains to create ZFNs and TALENs - a functional entity that is able to recognize its intended nucleic acid target through its engineered (ZFP or TALE) DNA binding domain and cause the DNA to be cut near the DNA binding site via the nuclease activity. See, *e.g.,* Kim et al. (1996) Proc Nat'lAcad Sci USA 93(3):1156-1160. More recently, such nucleases have been used for genome modification in a variety of organisms. *See,* for example, United States Patent Publications 20030232410; 20050208489; 20050026157; 20050064474; 20060188987; 20060063231; and International Publication WO 07/014275.

Thus, the methods and compositions described herein are broadly applicable and may involve any nuclease of interest. Non-limiting examples of nucleases include meganucleases, TALENs and zinc finger nucleases. The nuclease may comprise heterologous DNA-binding and cleavage domains (*e.g*., zinc finger nucleases; meganuclease DNA-binding domains with heterologous cleavage domains) or, alternatively, the DNA-binding domain of a naturally-occurring nuclease may be altered to bind to a selected target site (*e.g.,* a meganuclease that has been engineered to bind to site different than the cognate binding site).

In any of the nucleases described herein, the nuclease can comprise an engineered TALE DNA-binding domain and a nuclease domain (*e.g*., endonuclease and/or meganuclease domain), also referred to as TALENs. Methods and compositions for engineering these TALEN proteins for robust, site specific interaction with the target sequence of the user's choosing have been published (see U.S. Patent No. 8,586,526). In some embodiments, the TALEN comprises a endonuclease (*e.g*., FokI) cleavage domain or cleavage half-domain. In other embodiments, the TALE-nuclease is a mega TAL. These mega TAL nucleases are fusion proteins comprising a TALE DNA binding domain and a meganuclease cleavage domain. The meganuclease cleavage domain is active as a monomer and does not require dimerization for activity. (See Boissel et al., (2013) Nucl Acid Res: 1-13, doi: 10.1093/nar/gkt1224). In addition, the nuclease domain may also exhibit DNA-binding functionality.

In still further embodiments, the nuclease comprises a compact TALEN (cTALEN). These are single chain fusion proteins linking a TALE DNA binding domain to a TevI nuclease domain. The fusion protein can act as either a nickase localized by the TALE region, or can create a double strand break, depending upon where the TALE DNA binding domain is located with respect to the TevI nuclease domain (see Beurdeley et al (2013) Nat Comm: 1-8 DOI: 10.103 8/ncomms2782). Any TALENs may be used in combination with additional TALENs (*e.g*., one or more TALENs (cTALENs or FokI-TALENs) with one or more mega-TALs) or other DNA cleavage enzymes.

In certain embodiments, the nuclease comprises a meganuclease (homing endonuclease) or a portion thereof that exhibits cleavage activity. Naturally-occurring meganucleases recognize 15-40 base-pair cleavage sites and are commonly grouped into four families: the LAGLIDADG family, the GIY-YIG family, the His-Cyst box family and the HNH family. Exemplary homing endonucleases include I-*Sce*I, I*-Ceu*I*,* PI*-Psp*I*,* PI*-Sce,* I*-Sce*IV*,* I*-Csm*I*,* I*-Pan*I*,* I*-Sce*II*,* I*-Ppo*I*,* I-*Sce*III, I-*Cre*I, I*-Tev*I, I-*Tev*II and I-*Tev*III. Their recognition sequences are known. *See* also U.S. Patent No. 5,420,032; U.S. Patent No. 6,833,252; Belfort et al. (1997) Nucleic Acids Res. 25:3379-3388; Dujon et al. (1989) Gene 82:115-118; Perler et al. (1994) Nucleic Acids Res. 22, 1125-1127; Jasin (1996) Trends Genet. 12:224-228; Gimble et al. (1996) J. Mol. Biol. 263:163-180; Argast et al. (1998) J. Mol. Biol. 280:345-353 and the New England Biolabs catalogue.

DNA-binding domains from naturally-occurring meganucleases, primarily from the LAGLIDADG family, have been used to promote site-specific genome modification in plants, yeast, Drosophila, mammalian cells and mice, but this approach has been limited to the modification of either homologous genes that conserve the meganuclease recognition sequence (Monet et al. (1999), Biochem. Biophysics. Res. Common. 255: 88-93) or to pre-engineered genomes into which a recognition sequence has been introduced (Route et al. (1994), Mol. Cell. Biol. 14: 8096-106; Chilton et al. (2003), Plant Physiology. 133: 956-65; Puchta et al. (1996), Proc. Natl. Acad. Sci. USA 93: 5055-60; Rong et al. (2002), Genes Dev. 16: 1568-81; Gouble et al. (2006), J. Gene Med. 8(5):616-622). Accordingly, attempts have been made to engineer meganucleases to exhibit novel binding specificity at medically or biotechnologically relevant sites (Porteus et al. (2005), Nat. Biotechnol. 23: 967-73; Sussman et al. (2004), J Mol. Biol. 342: 31-41; Epinat et al. (2003), Nucleic Acids Res. 31: 2952-62; Chevalier et al. (2002) Molec. Cell 10:895-905; Epinat et al. (2003) Nucleic Acids Res. 31:2952-2962; Ashworth et al. (2006) Nature 441:656-659; Paques et al. (2007) Current Gene Therapy 7:49-66; U.S. Patent Publication Nos. 20070117128; 20060206949; 20060153826; 20060078552; and 20040002092). In addition, naturally-occurring or engineered DNA-binding domains from meganucleases can be operably linked with a cleavage domain from a heterologous nuclease (*e.g., Fok*I) and/or cleavage domains from meganucleases can be operably linked with a heterologous DNA-binding domain (*e.g*., ZFP or TALE).

In other embodiments, the nuclease is a zinc finger nuclease (ZFN) or TALE DNA binding domain-nuclease fusion (TALEN). ZFNs and TALENs comprise a DNA binding domain (zinc finger protein or TALE DNA binding domain) that has been engineered to bind to a target site in a gene of choice and cleavage domain or a cleavage half-domain (*e.g*., from a restriction and/or meganuclease as described herein).

As described in detail above, zinc finger binding domains and TALE DNA binding domains can be engineered to bind to a sequence of choice. *See,* for example, Beerli et al. (2002) Nature Biotechnol. 20:135-141; Pabo et al. (2001) Ann. Rev. Biochem. 70:313-340; Isalan et al. (2001) Nature Biotechnol. 19:656-660; Segal et al. (2001) Curr. Opin. Biotechnol. 12:632-637; Choo et al. (2000) Curr. Opin. Struct. Biol. 10:411-416. An engineered zinc finger binding domain or TALE protein can have a novel binding specificity, compared to a naturally-occurring protein. Engineering methods include, but are not limited to, rational design and various types of selection. Rational design includes, for example, using databases comprising triplet (or quadruplet) nucleotide sequences and individual zinc finger or TALE amino acid sequences, in which each triplet or quadruplet nucleotide sequence is associated with one or more amino acid sequences of zinc fingers or TALE repeat units which bind the particular triplet or quadruplet sequence. *See*, for example, U.S. Patents 6,453,242 and 6,534,261.

Selection of target sites; and methods for design and construction of fusion proteins (and polynucleotides encoding same) are known to those of skill in the art and described in detail in U.S. Patent Nos. 7,888,121 and 8,409,861.

In addition, as disclosed in these and other references, zinc finger domains, TALEs and/or multi-fingered zinc finger proteins may be linked together using any suitable linker sequences, including for example, linkers of 5 or more amino acids in length. (*e.g*., TGEKP (SEQ ID NO:3), TGGQRP (SEQ ID NO:4), TGQKP (SEQ ID NO:5), and/or TGSQKP (SEQ ID NO:6)). *See, e.g.,* U.S. Patent Nos. 6,479,626; 6,903,185; and 7,153,949 for exemplary linker sequences 6 or more amino acids in length. The proteins described herein may include any combination of suitable linkers between the individual zinc fingers of the protein. *See, also,* U.S. Provisional Patent Application No. 61/343,729.

Thus, nucleases such as ZFNs, TALENs and/or meganucleases can comprise any DNA-binding domain and any nuclease (cleavage) domain (cleavage domain, cleavage half-domain). As noted above, the cleavage domain may be heterologous to the DNA-binding domain, for example a zinc finger or TAL-effector DNA-binding domain and a cleavage domain from a nuclease or a meganuclease DNA-binding domain and cleavage domain from a different nuclease. Heterologous cleavage domains can be obtained from any endonuclease or exonuclease. Exemplary endonucleases from which a cleavage domain can be derived include, but are not limited to, restriction endonucleases and homing endonucleases. *See,* for example, 2002-2003 Catalogue, New England Biolabs, Beverly, MA; and Belfort et al. (1997) Nucleic Acids Res. 25:3379-3388. Additional enzymes which cleave DNA are known (*e.g.,* S1 Nuclease; mung bean nuclease; pancreatic DNase I; micrococcal nuclease; yeast HO endonuclease; *see* also Linn et al. (eds.) Nucleases, Cold Spring Harbor Laboratory Press,1993). One or more of these enzymes (or functional fragments thereof) can be used as a source of cleavage domains and cleavage half-domains.

Similarly, a cleavage half-domain can be derived from any nuclease or portion thereof, as set forth above, that requires dimerization for cleavage activity. In general, two fusion proteins are required for cleavage if the fusion proteins comprise cleavage half-domains. Alternatively, a single protein comprising two cleavage half-domains can be used. The two cleavage half-domains can be derived from the same endonuclease (or functional fragments thereof), or each cleavage half-domain can be derived from a different endonuclease (or functional fragments thereof). In addition, the target sites for the two fusion proteins are preferably disposed, with respect to each other, such that binding of the two fusion proteins to their respective target sites places the cleavage half-domains in a spatial orientation to each other that allows the cleavage half-domains to form a functional cleavage domain, *e.g*., by dimerizing. Thus, in certain embodiments, the near edges of the target sites are separated by 5-8 nucleotides or by 15-18 nucleotides. However any integral number of nucleotides or nucleotide pairs can intervene between two target sites (*e.g.,* from 2 to 50 nucleotide pairs or more). In general, the site of cleavage lies between the target sites.

Restriction endonucleases (restriction enzymes) are present in many species and are capable of sequence-specific binding to DNA (at a recognition site), and cleaving DNA at or near the site of binding. Certain restriction enzymes (*e.g*., Type IIS) cleave DNA at sites removed from the recognition site and have separable binding and cleavage domains. For example, the Type IIS enzyme *Fok* I catalyzes double-stranded cleavage of DNA, at 9 nucleotides from its recognition site on one strand and 13 nucleotides from its recognition site on the other. *See,* for example, US Patents 5,356,802; 5,436,150 and 5,487,994; as well as Li et al. (1992) Proc. Natl. Acad. Sci. USA 89:4275-4279; Li et al. (1993) Proc. Natl. Acad. Sci. USA 90:2764-2768; Kim et al. (1994a) Proc. Natl. Acad. Sci. USA 91:883-887; Kim et al. (1994b) J. Biol. Chem. 269:31,978-31,982. Thus, in one embodiment, fusion proteins comprise the cleavage domain (or cleavage half-domain) from at least one Type IIS restriction enzyme and one or more zinc finger binding domains, which may or may not be engineered.

An exemplary Type IIS restriction enzyme, whose cleavage domain is separable from the binding domain, is *Fok* I. This particular enzyme is active as a dimer. Bitinaite et al. (1998) Proc. Natl. Acad. Sci. USA 95: 10,570-10,575. Accordingly, for the purposes of the present disclosure, the portion of the *Fok* I enzyme used in the disclosed fusion proteins is considered a cleavage half-domain. Thus, for targeted double-stranded cleavage and/or targeted replacement of cellular sequences using zinc finger-*Fok* I fusions, two fusion proteins, each comprising a *Fok*I cleavage half-domain, can be used to reconstitute a catalytically active cleavage domain. Alternatively, a single polypeptide molecule containing a zinc finger binding domain and two *Fok* I cleavage half-domains can also be used. Parameters for targeted cleavage and targeted sequence alteration using zinc finger-*Fok* I fusions are provided elsewhere in this disclosure.

A cleavage domain or cleavage half-domain can be any portion of a protein that retains cleavage activity, or that retains the ability to multimerize (*e.g*., dimerize) to form a functional cleavage domain.

Exemplary Type IIS restriction enzymes are described in International Publication WO 07/014275. Additional restriction enzymes also contain separable binding and cleavage domains, and these are contemplated by the present disclosure. *See,* for example, Roberts et al. (2003) Nucleic Acids Res. 31:418-420.

In certain embodiments, the cleavage domain comprises one or more engineered cleavage half-domain (also referred to as dimerization domain mutants) that minimize or prevent homodimerization, as described, for example, in U.S. Patent Nos. 7,914,796; 8,034,598 and 8,623,618; and U.S. Patent Publication No. 20110201055. Amino acid residues at positions 446, 447, 479, 483, 484, 486, 487, 490, 491, 496, 498, 499, 500, 531, 534, 537, and 538 of *Fok* I are all targets for influencing dimerization of the *Fok*I cleavage half-domains.

Exemplary engineered cleavage half-domains of *Fok* I that form obligate heterodimers include a pair in which a first cleavage half-domain includes mutations at amino acid residues at positions 490 and 538 of *Fok* I and a second cleavage half-domain includes mutations at amino acid residues 486 and 499.

Thus, in one embodiment, a mutation at 490 replaces Glu (E) with Lys (K); the mutation at 538 replaces Iso (I) with Lys (K); the mutation at 486 replaced Gln (Q) with Glu (E); and the mutation at position 499 replaces Iso (I) with Lys (K). Specifically, the engineered cleavage half-domains described herein were prepared by mutating positions 490 (E→K) and 538 (I→K) in one cleavage half-domain to produce an engineered cleavage half-domain designated "E490K:I538K" and by mutating positions 486 (Q→E) and 499 (I→L) in another cleavage half-domain to produce an engineered cleavage half-domain designated "Q486E:I499L". The engineered cleavage half-domains described herein are obligate heterodimer mutants in which aberrant cleavage is minimized or abolished. *See, e.g.,* U.S. Patent Publication No. 2008/0131962. In certain embodiments, the engineered cleavage half-domain comprises mutations at positions 486, 499 and 496 (numbered relative to wild-type *Fok*I), for instance mutations that replace the wild type Gln (Q) residue at position 486 with a Glu (E) residue, the wild type Iso (I) residue at position 499 with a Leu (L) residue and the wild-type Asn (N) residue at position 496 with an Asp (D) or Glu (E) residue (also referred to as a "ELD" and "ELE" domains, respectively). In other embodiments, the engineered cleavage half-domain comprises mutations at positions 490, 538 and 537 (numbered relative to wild-type *Fok*I), for instance mutations that replace the wild type Glu (E) residue at position 490 with a Lys (K) residue, the wild type Iso (I) residue at position 538 with a Lys (K) residue, and the wild-type His (H) residue at position 537 with a Lys (K) residue or a Arg (R) residue (also referred to as "KKK" and "KKR" domains, respectively). In other embodiments, the engineered cleavage half-domain comprises mutations at positions 490 and 537 (numbered relative to wild-type FokI), for instance mutations that replace the wild type Glu (E) residue at position 490 with a Lys (K) residue and the wild-type His (H) residue at position 537 with a Lys (K) residue or a Arg (R) residue (also referred to as "KIK" and "KIR" domains, respectively). ((See US Patent Publication No. 20110201055).

Engineered cleavage half-domains described herein can be prepared using any suitable method, for example, by site-directed mutagenesis of wild-type cleavage half-domains (*Fok*I) as described in U.S. Patent Nos. 7,914,796; 8,034,598 and 8,623,618; and U.S. Patent Publication No. 20110201055.

Alternatively, nucleases may be assembled *in vivo* at the nucleic acid target site using so-called "split-enzyme" technology (*see e.g.* U.S. Patent Publication No. 20090068164). Components of such split enzymes may be expressed either on separate expression constructs, or can be linked in one open reading frame where the individual components are separated, for example, by a self-cleaving 2A peptide or IRES sequence. Components may be individual zinc finger binding domains or domains of a meganuclease nucleic acid binding domain.

Nucleases (*e.g*., ZFNs and/or TALENs) can be screened for activity prior to use, for example in a yeast-based chromosomal system as described in WO 2009/042163 and 20090068164. Nuclease expression constructs can be readily designed using methods known in the art. See, *e.g.,* United States Patent Publications 20030232410;20050208489;20050026157;20050064474;20060188987; 20060063231; and International Publication WO 07/014275. Expression of the nuclease may be under the control of a constitutive promoter or an inducible promoter, for example the galactokinase promoter which is activated (de-repressed) in the presence of raffinose and/or galactose and repressed in presence of glucose.

In certain embodiments, the nuclease comprises a CRISPR/Cas system. The CRISPR (clustered regularly interspaced short palindromic repeats) locus, which encodes RNA components of the system, and the cas (CRISPR-associated) locus, which encodes proteins (Jansen et al., 2002. Mol. Microbiol. 43: 1565-1575; Makarova et al., 2002. Nucleic Acids Res. 30: 482-496; Makarova et al., 2006. Biol. Direct 1: 7; Haft et al., 2005. PLoS Comput. Biol. 1: e60) make up the gene sequences of the CRISPR/Cas nuclease system. CRISPR loci in microbial hosts contain a combination of CRISPR-associated (Cas) genes as well as non-coding RNA elements capable of programming the specificity of the CRISPR-mediated nucleic acid cleavage.

The Type II CRISPR is one of the most well characterized systems and carries out targeted DNA double-strand break in four sequential steps. First, two non-coding RNA, the pre-crRNA array and tracrRNA, are transcribed from the CRISPR locus. Second, tracrRNA hybridizes to the repeat regions of the pre-crRNA and mediates the processing of pre-crRNA into mature crRNAs containing individual spacer sequences. Third, the mature crRNA:tracrRNA complex directs Cas9 to the target DNA via Watson-Crick base-pairing between the spacer on the crRNA and the protospacer on the target DNA next to the protospacer adjacent motif (PAM), an additional requirement for target recognition. Finally, Cas9 mediates cleavage of target DNA to create a double-stranded break within the protospacer. Activity of the CRISPR/Cas system comprises of three steps: (i) insertion of alien DNA sequences into the CRISPR array to prevent future attacks, in a process called 'adaptation', (ii) expression of the relevant proteins, as well as expression and processing of the array, followed by (iii) RNA-mediated interference with the alien nucleic acid. Thus, in the bacterial cell, several of the so-called 'Cas' proteins are involved with the natural function of the CRISPR/Cas system and serve roles in functions such as insertion of the alien DNA etc.

In certain embodiments, Cas protein may be a "functional derivative" of a naturally occurring Cas protein. A "functional derivative" of a native sequence polypeptide is a compound having a qualitative biological property in common with a native sequence polypeptide. "Functional derivatives" include, but are not limited to, fragments of a native sequence and derivatives of a native sequence polypeptide and its fragments, provided that they have a biological activity in common with a corresponding native sequence polypeptide. A biological activity contemplated herein is the ability of the functional derivative to hydrolyze a DNA substrate into fragments. The term "derivative" encompasses both amino acid sequence variants of polypeptide, covalent modifications, and fusions thereof. Suitable derivatives of a Cas polypeptide or a fragment thereof include but are not limited to mutants, fusions, covalent modifications of Cas protein or a fragment thereof. Cas protein, which includes Cas protein or a fragment thereof, as well as derivatives of Cas protein or a fragment thereof, may be obtainable from a cell or synthesized chemically or by a combination of these two procedures. The cell may be a cell that naturally produces Cas protein, or a cell that naturally produces Cas protein and is genetically engineered to produce the endogenous Cas protein at a higher expression level or to produce a Cas protein from an exogenously introduced nucleic acid, which nucleic acid encodes a Cas that is same or different from the endogenous Cas. In some case, the cell does not naturally produce Cas protein and is genetically engineered to produce a Cas protein.

Exemplary CRISPR/Cas nuclease systems targeted to HLA and other genes are disclosed for example, in U.S. Provisional Application No. 61/823,689.

### Delivery

The proteins (*e.g*., nucleases and non-classic HLA molecules), polynucleotides encoding same and compositions comprising the proteins and/or polynucleotides described herein may be delivered to a target cell by any suitable means, including, for example, by injection of the protein and/or mRNA.

Suitable cells include but not limited to eukaryotic and prokaryotic cells and/or cell lines. Non-limiting examples of such cells or cell lines generated from such cells include T-cells, COS, CHO (*e.g.,* CHO-S, CHO-K1, CHO-DG44, CHO-DUXB11, CHO-DUKX, CHOK1SV), VERO, MDCK, WI38, V79, B14AF28-G3, BHK, HaK, NS0, SP2/0-Agl4, HeLa, HEK293 (*e.g*., HEK293-F, HEK293-H, HEK293-T), and perC6 cells as well as insect cells such as *Spodoptera fugiperda* (Sf), or fungal cells such as *Saccharomyces, Pichia* and *Schizosaccharomyces.* In certain embodiments, the cell line is a CHO-K1, MDCK or HEK293 cell line. Suitable cells also include stem cells such as, by way of example, embryonic stem cells, induced pluripotent stem cells (iPS cells), hematopoietic stem cells, neuronal stem cells and mesenchymal stem cells.

Methods of delivering proteins comprising DNA-binding domains as described herein are described, for example, in U.S. Patent Nos. 6,453,242; 6,503,717; 6,534,261; 6,599,692; 6,607,882; 6,689,558; 6,824,978; 6,933,113; 6,979,539; 7,013,219; and 7,163,824.

DNA binding domains and fusion proteins comprising these DNA binding domains as described herein may also be delivered using vectors containing sequences encoding one or more of the DNA-binding protein(s). Additionally, additional nucleic acids (*e.g*., donors and/or sequences encoding non-classic HLA proteins) also may be delivered via these vectors. Any vector systems may be used including, but not limited to, plasmid vectors, retroviral vectors, lentiviral vectors, adenovirus vectors, poxvirus vectors; herpesvirus vectors and adeno-associated virus vectors, etc. *See, also,* U.S. Patent Nos. 6,534,261; 6,607,882; 6,824,978; 6,933,113; 6,979,539; 7,013,219; and 7,163,824. Furthermore, it will be apparent that any of these vectors may comprise one or more DNA-binding protein-encoding sequences and/or additional nucleic acids as appropriate. Thus, when one or more DNA-binding proteins as described herein are introduced into the cell, and additional DNAs as appropriate, they may be carried on the same vector or on different vectors. When multiple vectors are used, each vector may comprise a sequence encoding one or multiple DNA-binding proteins and additional nucleic acids as desired.

Conventional viral and non-viral based gene transfer methods can be used to introduce nucleic acids encoding engineered DNA-binding proteins in cells (*e.g*., mammalian cells) and target tissues and to co-introduce additional nucleotide sequences as desired. Such methods can also be used to administer nucleic acids (*e.g*., encoding DNA-binding proteins, donors and/or non-classic HLA proteins) to cells *in vitro.* In certain embodiments, nucleic acids are administered for *in vivo* or *ex vivo* gene therapy uses. Non-viral vector delivery systems include DNA plasmids, naked nucleic acid, and nucleic acid complexed with a delivery vehicle such as a liposome or poloxamer. Viral vector delivery systems include DNA and RNA viruses, which have either episomal or integrated genomes after delivery to the cell. For a review of gene therapy procedures, see Anderson, Science 256:808-813 (1992); Nabel & Felgner, TIBTECH 11:211-217 (1993); Mitani & Caskey, TIBTECH 11:162-166 (1993); Dillon, TIBTECH 11:167-175 (1993); Miller, Nature 357:455-460 (1992); Van Brunt, Biotechnology 6(10):1149-1154 (1988); Vigne, Restorative Neurology and Neuroscience 8:35-36 (1995); Kremer & Perricaudet, British Medical Bulletin 51(1):31-44 (1995); Haddada et al., in Current Topics in Microbiology and Immunology Doerfler and Böhm (eds.) (1995); and Yu et al., Gene Therapy 1:13-26 (1994).

Methods of non-viral delivery of nucleic acids include electroporation, lipofection, microinjection, biolistics, virosomes, liposomes, immunoliposomes, polycation or lipid:nucleic acid conjugates, naked DNA, mRNA, artificial virions, and agent-enhanced uptake of DNA. Sonoporation using, *e.g*., the Sonitron 2000 system (Rich-Mar) can also be used for delivery of nucleic acids. In a preferred embodiment, one or more nucleic acids are delivered as mRNA. Also preferred is the use of capped mRNAs to increase translational efficiency and/or mRNA stability. Especially preferred are ARCA (anti-reverse cap analog) caps or variants thereof. See U.S. Patent Nos. 7,074,596 and 8,153,773.

Additional exemplary nucleic acid delivery systems include those provided by Amaxa Biosystems (Cologne, Germany), Maxcyte, Inc. (Rockville, Maryland), BTX Molecular Delivery Systems (Holliston, MA) and Copernicus Therapeutics Inc, (*see* for example US6008336). Lipofection is described in *e.g.,* US 5,049,386, US 4,946,787; and US 4,897,355) and lipofection reagents are sold commercially (*e.g*., Transfectam™, Lipofectin™, and Lipofectamine™ RNAiMAX). Cationic and neutral lipids that are suitable for efficient receptor-recognition lipofection of polynucleotides include those of Felgner, WO 91/17424, WO 91/16024. Delivery can be to cells (*ex vivo* administration) or target tissues (*in vivo* administration).

The preparation of lipid:nucleic acid complexes, including targeted liposomes such as immunolipid complexes, is well known to one of skill in the art (*see, e.g.,* Crystal, Science 270:404-410 (1995); Blaese et al., Cancer Gene Ther. 2:291-297 (1995); Behr et al., Bioconjugate Chem. 5:382-389 (1994); Remy et al, Bioconjugate Chem. 5:647-654 (1994); Gao et al., Gene Therapy 2:710-722 (1995); Ahmad et al., Cancer Res. 52:4817-4820 (1992); U.S. Pat. Nos. 4,186,183, 4,217,344, 4,235,871, 4,261,975, 4,485,054, 4,501,728, 4,774,085, 4,837,028, and 4,946,787).

Additional methods of delivery include the use of packaging the nucleic acids to be delivered into EnGeneIC delivery vehicles (EDVs). These EDVs are specifically delivered to target tissues using bispecific antibodies where one arm of the antibody has specificity for the target tissue and the other has specificity for the EDV. The antibody brings the EDVs to the target cell surface and then the EDV is brought into the cell by endocytosis. Once in the cell, the contents are released *(see* MacDiarmid et al (2009) Nature Biotechnology vol 27(7) p. 643).

The use of RNA or DNA viral based systems for the delivery of nucleic acids encoding engineered DNA-binding proteins, non-classic HLA-molecules and/or other donors as desired takes advantage of highly evolved processes for targeting a virus to specific cells in the body and trafficking the viral payload to the nucleus. Viral vectors can be administered directly to patients (*in vivo*) or they can be used to treat cells *in vitro* and the modified cells are administered to patients (*ex vivo*). Conventional viral based systems for the delivery of nucleic acids include, but are not limited to, retroviral, lentivirus, adenoviral, adeno-associated, vaccinia and herpes simplex virus vectors for gene transfer. Integration in the host genome is possible with the retrovirus, lentivirus, and adeno-associated virus gene transfer methods, often resulting in long term expression of the inserted transgene. Additionally, high transduction efficiencies have been observed in many different cell types and target tissues.

The tropism of a retrovirus can be altered by incorporating foreign envelope proteins, expanding the potential target population of target cells. Lentiviral vectors are retroviral vectors that are able to transduce or infect non-dividing cells and typically produce high viral titers. Selection of a retroviral gene transfer system depends on the target tissue. Retroviral vectors are comprised of cis-acting long terminal repeats with packaging capacity for up to 6-10 kb of foreign sequence. The minimum cis-acting LTRs are sufficient for replication and packaging of the vectors, which are then used to integrate the therapeutic gene into the target cell to provide permanent transgene expression. Widely used retroviral vectors include those based upon murine leukemia virus (MuLV), gibbon ape leukemia virus (GaLV), Simian Immunodeficiency virus (SIV), human immunodeficiency virus (HIV), and combinations thereof (*see, e.g.,* Buchscher et al., J. Virol. 66:2731-2739 (1992); Johann et al., J. Virol. 66:1635-1640 (1992); Sommerfelt et al., Virol. 176:58-59 (1990); Wilson et al., J. Virol. 63:2374-2378 (1989); Miller et al., J. Virol. 65:2220-2224 (1991); PCT/US94/05700).

In applications in which transient expression is preferred, adenoviral based systems can be used. Adenoviral based vectors are capable of very high transduction efficiency in many cell types and do not require cell division. With such vectors, high titer and high levels of expression have been obtained. This vector can be produced in large quantities in a relatively simple system. Adeno-associated virus ("AAV") vectors are also used to transduce cells with target nucleic acids, *e.g.,* in the *in vitro* production of nucleic acids and peptides, and for *in vivo* and *ex vivo* gene therapy procedures (*see, e.g.,* West et al., Virology 160:38-47 (1987); U.S. Patent No. 4,797,368; WO 93/24641; Kotin, Human Gene Therapy 5:793-801 (1994); Muzyczka, J. Clin. Invest. 94:1351 (1994). Construction of recombinant AAV vectors are described in a number of publications, including U.S. Pat. No. 5,173,414; Tratschin et al., Mol. Cell. Biol. 5:3251-3260 (1985); Tratschin, et al., Mol. Cell. Biol. 4:2072-2081 (1984); Hermonat & Muzyczka, PNAS 81:6466-6470 (1984); and Samulski et al., J. Virol. 63:03822-3828 (1989).

At least six viral vector approaches are currently available for gene transfer in clinical trials, which utilize approaches that involve complementation of defective vectors by genes inserted into helper cell lines to generate the transducing agent.

pLASN and MFG-S are examples of retroviral vectors that have been used in clinical trials (Dunbar et al., Blood 85:3048-305 (1995); Kohn et al., Nat. Med. 1:1017-102 (1995); Malech et al., PNAS 94:22 12133-12138 (1997)). PA317/pLASN was the first therapeutic vector used in a gene therapy trial. (Blaese et al., Science 270:475-480 (1995)). Transduction efficiencies of 50% or greater have been observed for MFG-S packaged vectors. (Ellem et al., Immunol Immunother. 44(1):10-20 (1997); Dranoff et al., Hum. Gene Ther. 1:111-2 (1997).

Recombinant adeno-associated virus vectors (rAAV) are a promising alternative gene delivery systems based on the defective and nonpathogenic parvovirus adeno-associated type 2 virus. All vectors are derived from a plasmid that retains only the AAV 145 bp inverted terminal repeats flanking the transgene expression cassette. Efficient gene transfer and stable transgene delivery due to integration into the genomes of the transduced cell are key features for this vector system. (Wagner et al., Lancet 351:9117 1702-3 (1998), Kearns et al., Gene Ther. 9:748-55 (1996)). Other AAV serotypes, including AAV1, AAV3, AAV4, AAV5, AAV6, AAV8, AAV8.2, AAV9 and AAVrh10 and pseudotyped AAV such as AAV2/8, AAV2/5 and AAV2/6 can also be used in accordance with the present invention.

Replication-deficient recombinant adenoviral vectors (Ad) can be produced at high titer and readily infect a number of different cell types. Most adenovirus vectors are engineered such that a transgene replaces the Ad E1a, E1b, and/or E3 genes; subsequently the replication defective vector is propagated in human 293 cells that supply deleted gene function in *trans.* Ad vectors can transduce multiple types of tissues *in vivo,* including nondividing, differentiated cells such as those found in liver, kidney and muscle. Conventional Ad vectors have a large carrying capacity. An example of the use of an Ad vector in a clinical trial involved polynucleotide therapy for antitumor immunization with intramuscular injection (Sterman et al., Hum. Gene Ther. 7:1083-9 (1998)). Additional examples of the use of adenovirus vectors for gene transfer in clinical trials include Rosenecker et al., Infection 24:1 5-10 (1996); Sterman et al., Hum. Gene Ther. 9:7 1083-1089 (1998); Welsh et al., Hum. Gene Ther. 2:205-18 (1995); Alvarez et al., Hum. Gene Ther. 5:597-613 (1997); Topf et al., Gene Ther. 5:507-513 (1998); Sterman et al., Hum. Gene Ther. 7:1083-1089 (1998).

Packaging cells are used to form virus particles that are capable of infecting a host cell. Such cells include 293 cells, which package adenovirus, and ψ2 cells or PA317 cells, which package retrovirus. Viral vectors used in gene therapy are usually generated by a producer cell line that packages a nucleic acid vector into a viral particle. The vectors typically contain the minimal viral sequences required for packaging and subsequent integration into a host (if applicable), other viral sequences being replaced by an expression cassette encoding the protein to be expressed. The missing viral functions are supplied in *trans* by the packaging cell line. For example, AAV vectors used in gene therapy typically only possess inverted terminal repeat (ITR) sequences from the AAV genome which are required for packaging and integration into the host genome. Viral DNA is packaged in a cell line, which contains a helper plasmid encoding the other AAV genes, namely *rep* and *cap*, but lacking ITR sequences. The cell line is also infected with adenovirus as a helper. The helper virus promotes replication of the AAV vector and expression of AAV genes from the helper plasmid. The helper plasmid is not packaged in significant amounts due to a lack of ITR sequences. Contamination with adenovirus can be reduced by, *e.g.,* heat treatment to which adenovirus is more sensitive than AAV.

In many gene therapy applications, it is desirable that the gene therapy vector be delivered with a high degree of specificity to a particular tissue type. Accordingly, a viral vector can be modified to have specificity for a given cell type by expressing a ligand as a fusion protein with a viral coat protein on the outer surface of the virus. The ligand is chosen to have affinity for a receptor known to be present on the cell type of interest. For example, Han et al., Proc. Natl. Acad. Sci. USA 92:9747-9751 (1995), reported that Moloney murine leukemia virus can be modified to express human heregulin fused to gp70, and the recombinant virus infects certain human breast cancer cells expressing human epidermal growth factor receptor. This principle can be extended to other virus-target cell pairs, in which the target cell expresses a receptor and the virus expresses a fusion protein comprising a ligand for the cell-surface receptor. For example, filamentous phage can be engineered to display antibody fragments (*e.g*., FAB or Fv) having specific binding affinity for virtually any chosen cellular receptor. Although the above description applies primarily to viral vectors, the same principles can be applied to nonviral vectors. Such vectors can be engineered to contain specific uptake sequences which favor uptake by specific target cells.

Gene therapy vectors can be delivered *in vivo* by administration to an individual patient, typically by systemic administration (*e.g*., intravenous, intraperitoneal, intramuscular, subdermal, or intracranial infusion) or topical application, as described below. Alternatively, vectors can be delivered to cells *ex vivo*, such as cells explanted from an individual patient (*e.g.,* lymphocytes, bone marrow aspirates, tissue biopsy) or universal donor hematopoietic stem cells, followed by re-implantation of the cells into a patient, usually after selection for cells which have incorporated the vector.

*Ex vivo* cell transfection for diagnostics, research, transplant or for gene therapy (*e.g*., via re-infusion of the transfected cells into the host organism) is well known to those of skill in the art. In a preferred embodiment, cells are isolated from the subject organism, transfected with a DNA-binding proteins nucleic acid (gene or cDNA), and re-infused back into the subject organism (*e.g*., patient). Various cell types suitable for *ex vivo* transfection are well known to those of skill in the art (*see, e.g.,* Freshney et al., Culture of Animal Cells, A Manual of Basic Technique (3rd ed. 1994)) and the references cited therein for a discussion of how to isolate and culture cells from patients).

In one embodiment, stem cells are used in *ex vivo* procedures for cell transfection and gene therapy. The advantage to using stem cells is that they can be differentiated into other cell types *in vitro,* or can be introduced into a mammal (such as the donor of the cells) where they will engraft in the bone marrow. Methods for differentiating CD34+ cells *in vitro* into clinically important immune cell types using cytokines such a GM-CSF, IFN-γ and TNF-α are known (*see* Inaba et al., J. Exp. Med. 176:1693-1702 (1992)).

Stem cells are isolated for transduction and differentiation using known methods. For example, stem cells are isolated from bone marrow cells by panning the bone marrow cells with antibodies which bind unwanted cells, such as CD4+ and CD8+ (T cells), CD45+ (panB cells), GR-1 (granulocytes), and lad (differentiated antigen presenting cells) (*see* Inaba et al., J. Exp. Med. 176:1693-1702 (1992)).

Stem cells that have been modified may also be used in some embodiments. For example, neuronal stem cells that have been made resistant to apoptosis may be used as therapeutic compositions where the stem cells also contain the ZFP TFs of the invention. Resistance to apoptosis may come about, for example, by knocking out BAX and/or BAK using BAX- or BAK-specific ZFNs (see, US patent application no. 12/456,043) in the stem cells, or those that are disrupted in a caspase, again using caspase-6 specific ZFNs for example. These cells can be transfected with the ZFP TFs that are known to regulate HLA.

Vectors (*e.g*., retroviruses, adenoviruses, liposomes, etc.) containing therapeutic DNA-binding proteins (or nucleic acids encoding these proteins) can also be administered directly to an organism for transduction of cells *in vivo.* Alternatively, naked DNA can be administered. Administration is by any of the routes normally used for introducing a molecule into ultimate contact with blood or tissue cells including, but not limited to, injection, infusion, topical application and electroporation. Suitable methods of administering such nucleic acids are available and well known to those of skill in the art, and, although more than one route can be used to administer a particular composition, a particular route can often provide a more immediate and more effective reaction than another route.

Methods for introduction of DNA into hematopoietic stem cells are disclosed, for example, in U.S. Patent No. 5,928,638. Vectors useful for introduction of transgenes into hematopoietic stem cells, *e.g.,* CD34⁺ cells, include adenovirus Type 35.

Vectors suitable for introduction of transgenes into immune cells (*e.g.,* T-cells) include non-integrating lentivirus vectors. *See,* for example, Ory et al. (1996) Proc. Natl. Acad. Sci. USA 93:11382-11388; Dull et al. (1998) J. Virol. 72:8463-8471; Zuffery et al. (1998) J Virol. 72:9873-9880; Follenzi et al. (2000) Nature Genetics 25:217-222.

Pharmaceutically acceptable carriers are determined in part by the particular composition being administered, as well as by the particular method used to administer the composition. Accordingly, there is a wide variety of suitable formulations of pharmaceutical compositions available, as described below (*see, e.g.,* Remington's Pharmaceutical Sciences, 17th ed., 1989).

As noted above, the disclosed methods and compositions can be used in any type of cell including, but not limited to, prokaryotic cells, fungal cells, Archaeal cells, plant cells, insect cells, animal cells, vertebrate cells, mammalian cells and human cells, including T-cells and stem cells of any type. Suitable cell lines for protein expression are known to those of skill in the art and include, but are not limited to COS, CHO (*e.g.,* CHO-S, CHO-K1, CHO-DG44, CHO-DUXB11), VERO, MDCK, WI38, V79, B14AF28-G3, BHK, HaK, NSO, SP2/0-Ag14, HeLa, HEK293 (*e.g.,* HEK293-F, HEK293-H, HEK293-T), perC6, insect cells such as *Spodoptera fugiperda* (Sf), and fungal cells such as *Saccharomyces, Pichia* and *Schizosaccharomyces.* Progeny, variants and derivatives of these cell lines can also be used.

### Applications

The disclosed compositions and methods can be used for any application in which it is desired to modulate HLA expression and/or functionality, including but not limited to, therapeutic and research applications in which HLA modulation is desirable.

Diseases and conditions which are tied to HLA include Addison's disease, ankylosing spondylitis, Behçet's disease, Buerger's disease, celiac disease, chronic active hepatitis, Graves' disease, juvenile rheumatoid arthritis, psoriasis, psoriatic arthritis, rheumatoid arthritis, Sjögren syndrome, and lupus erythematosus, among others. In addition, modification of a HLA gene may be useful in conjunction with other genetic modifications of a cell of interest. For example, modification of a target cell such as a CTL with a chimeric antigen receptor to change the CTL's specificity may be combined with HLA modification *ex vivo* as described herein in order to develop a cell therapeutic that may be used in most any patient in need thereof.

In addition, the materials and methods of the invention can be used in the treatment, prevention or amelioration of graft-versus-host-disease. Graft-versus-host disease (GVHD) is a common complication when allogenic T-cells (*e.g.,* bone marrow and/or blood transfusion) are administered to a patient. The functional immune cells in the infused material recognize the recipient as "foreign" and mount an immunologic attack. By modulating HLA and/or TCR expression in allogenic T cells, "off the shelf' T cells (*e.g.,* CD 19-specific T-cells) can be administered on demand as "drugs" because the risk of GVHD is reduced or eliminated and, in addition, provision of non-classic HLA molecules reduces or eliminates NK-mediated lysis of the modified cells.

Methods and compositions also include stem cell compositions wherein one or more classic HLA genes within the stem cells has been inactivated and one or more non-classic HLA molecules activated. For example, HLA-modified hematopoietic stem cells can be introduced into a patient following bone marrow ablation. These altered HSC would allow the re-colonization of the patient without loss of the graft due to rejection and/or NK-mediated cell lysis. The introduced cells may also have other alterations to help during subsequent therapy (*e.g*., chemotherapy resistance) to treat the underlying disease.

The methods and compositions of the invention are also useful for the development of HLA modified platelets, for example for use as therapeutics. Thus, HLA modified platelets may be used to treat thrombocytopenic disorders such as idiopathic thrombocytopenic purpura, thrombotic thrombocytopenic purpura and drug-induced thrombocytopenic purpura (*e.g*. heparin-induced thrombocytopenia). Other platelet disorders that may be treated with the HLA modified platelets of the invention include Gaucher's disease, aplastic anemia, Onyalai, fetomaternal alloimmune thrombocytopenia, HELLP syndrome, cancer and side effects from some chemotherapeutic agents. The HLA modified platelets also have use in as an "off the shelf' therapy in emergency room situations with trauma patients.

The methods and compositions of the invention can be used in xenotransplantation. Specifically, by way of example only, pig organs can be used for transplantation into humans wherein the porcine MHC genes have been deleted and/or replaced with human HLA genes. Strains of pigs can be developed (from pig embryos that have had HLA targeting ZFNs encoded by mRNAs injected into them such that the endogenous MHC genes are disrupted, or from somatic cell nuclear transfer into pig embryos using nuclei of cells that have been successfully had their HLA genes targeted) that contain these useful genetic mutations, and these animals may be grown for eventual organ harvest. This will prevent rejection of these organs in humans and increase the chances for successful transplantation.

The methods and compositions of the invention are also useful for the design and implementation of *in vitro* and *in vivo* models, for example, animal models of HLA or other disorders, which allows for the study of these disorders.

### EXAMPLES

### Example 1: Materials and Methods

### ZFNs

HLA-A-binding ZFNs containing 5 or 6 fingers were designed and assembled using an established archive of pre-validated 2-finger and 1-finger modules as described in U.S. Patent Publication No. 20120060230. Exemplary ZFNs that may be used are shown below in Table 1. The first column in this table is an internal reference name (number) for a ZFP and corresponds to the same name in column 1 of Table 2. "F" refers to the finger and the number following "F" refers which zinc finger (*e.g.,* "F1" refers to finger 1).

**Table 1: Zinc finger proteins**

| Target | SBS # | Design | | | | | |
|---|---|---|---|---|---|---|---|
| **Class** I | | F1 | F2 | F3 | F4 | F5 | F6 |
| **HLA A2** | 18889 | QSSHLTR (SEQ ID NO:11) | RSDHLTT (SEQ ID NO:12) | RSDTLSQ (SEQ ID NO:13) | RSADLSR (SEQ ID NO:14) | QSSDLSR (SEQ ID NO:15) | RSDALTQ (SEQ ID NO:16) |
| **HLA A2** | 18881 | QKTHLAK (SEQ ID NO:17) | RSDTLSN (SEQ ID NO:18) | RKDVRIT (SEQ ID NO:19) | RSDHLST (SEQ ID NO:20) | DSSARKK (SEQ ID NO:21) | NA |
| **HLA A2** | 24859 | QNAHRKT (SEQ ID NO:22) | RSDSLLR (SEQ ID NO:23) | RNDDRKK (SEQ ID NO:24) | RSDHLST (SEQ ID NO:20) | DSSARKK (SEQ ID NO:21) | NA |
| **HLA A3** | 25191 | DRSHLSR (SEQ ID NO:25) | RSDDLTR (SEQ ID NO:2 6) | DRSDLSR (SEQ ID NO:27) | QSGHLSR (SEQ ID NO:28) | NA | NA |
| **HLA A3** | 25190 | DRSALSR (SEQ ID NO:29) | QSSDLRR (SEQ ID NO:30) | DRSALSR (SEQ ID NO:29) | DRSHLAR (SEQ ID NO:31) | RSDDLSK (SEQ ID NO:32) | DRSHLAR (SEQ ID NO:31) |

The sequence for the target sites of exemplary HLA-A binding proteins are disclosed in Table 2. Table 2 shows target sequences for the indicated zinc finger proteins. Nucleotides in the target site that are contacted by the ZFP recognition helices are indicated in uppercase letters; non-contacted nucleotides indicated in lowercase.

**Table 2: HLA-A Zinc finger target sites**

| Target | SBS # | Target site |
|---|---|---|
| **Class I** | | |
| **HLA A2** | 18889 | gtATGGCTGCGACGTGGGGTcggacggg_(SEQ ID NO:34) |
| **HLA A2** | 18881 | ttATCTGGATGGTGTGAgaacctggccc_(SEQ ID NO:35) |
| **HLA A2** | 24859 | tcCTCTGGACGGTGTGAgaacctggccc_(SEQ ID NO:36) |
| **HLA A3** | 25191 | atGGAGCCGCGGGCgccgtggatagagc_(SEQ ID NO:37) |
| **HLA A3** | 25190 | ctGGCTCGcGGCGTCGCTGTCgaaccgc_(SEQ ID NO:38) |

### Cell Culture

HEK293 cells were maintained in Dulbecco's modified Eagle's medium (Lonza, Basel, Switzerland) supplemented with 10% heat-inactivated fetal bovine serum (FBS: Lonza) and 2 mmol/L L-glutamine (Glutamax-1: Invitrogen, Carlsbad, CA). EBV-LCL, 721.221 and EL-4 cell lines were maintained in RPMI 1640 (Lonza) supplemented with 10% heat-inactivated FBS and 2 mmol/L L-glutamine. Identity of these cell lines was confirmed by STR DNA fingerprinting. CD8+ CTL clones specific for mHAgs were: clone 7A7 (Brickner et al. (2006) Blood 107(9):3779-3786) recognizing peptide RVWDLPGVLK (SEQ ID NO:1) encoded by PANE1 transcripts in the context of HLA-A*0301 and clone GAS2B3-5 (Tykodi et al. (2008) Clin Cancer Res. 14(16):5260-5269) recognizing HLA-A*0201-restricted CIPPDSLLFPA (SEQ ID NO:2, alternative open reading frame of NM_199250.1) peptide from ORF +2/48 in C19ORF48. CTL clones were thawed one day before the ⁵¹Chromium release assay, and maintained in RPMI 1640 supplemented with 10% human albumin serum, 2 mmol/L L-glutamine, 20 ng/mL of IL-15 (PeproTech, Rocky Hill, NJ), and 20 IU/mL of IL-2 (Chiron, Emeryville, CA).

### Activation of primary T cells by OKT3-loaded artificial antigen presenting cells (aAPC)

T cells (CAR^{neg}) were activated for sustained proliferation by crosslinking CD3 *in vitro* by stimulating PBMC with OKT3 (eBioscience, San Diego, CA) pre-loaded onto aAPC (clone #4: K562 cells genetically modified to stably co-express CD19, CD64, CD86, CD137L, and a membrane-bound mutein of interleukin IL-15 synchronously expressed with EGFP17 (see, O'Connor et al. (2012) Sci Rep 2:249; Manuri et al. (2010) Hum Gene Ther 21(4):427-437)) at a ratio of 1:1 (T cells : γ-irradiated (100Gy) aAPC) in RPMI 1640 supplemented with 2 mmol/L L-glutamine and 10% FBS with 50 IU/mL of IL-2 (added every other day, beginning the day after addition of aAPC). OKT3-loaded aAPC were re-added every 14 days to sustain T-cell proliferation.

### Generation of genetically modified CD19-specific CAR+ T cells and propagation on CD19+ aAPC

Our approach to manufacture clinical-grade CAR+ T cells was adapted to generate CD19-specific T cells. (See, *e.g.,* Singh et al. (2008) Cancer Res. 68(8):2961-2971). DNA plasmids coding for SB transposon CD19RCD28 and SB hyperactive transposase SB11 were simultaneously electro-transferred (Human T-Cell Nucleofector solution, program U-014) using a Nucleofector II device (Lonza) into T cells derived from PBMC. A population of CAR+ T cells was selectively numerically expanded by adding on the day of electroporation, and re-adding every 14 days (at 1 : 2 T cell : aAPC ratio) γ-irradiated (100 Gy) aAPC (clone #4 without OKT3 loading) in the presence of 50 IU/mL of IL-2 (added every other day, beginning the day after addition of aAPC).

### In vitro transcription of messenger RNA

*In vitro*-transcribed mRNA species were prepared as previously described in Torikai et al. (2012) Blood 119(24):5697-5705. In brief, the DNA template plasmids coding for ZFN-L and ZFN-R were linearized with XhoI. After *in vitro* transcription (RiboMAX™ Large Scale RNA Production System-T7, Promega, Madison, WI) and capping (ARCA cap analog, Ambion, Austin, TX) according to manufacturers' instructions, poly-adenines were added using the poly A tailing kit (Ambion). The integrity of the mRNA species was validated on a denaturing 1% agarose gel with 3-(N-morpholino) propanesulphonic acid (MOPS) buffer and concentration was determined by spectrophotometer (BioRad, Hercules, CA) at OD260.The mRNA was vialed and stored at -80°C for one-time use.

### Electro-transfer of DNA plasmids and mRNA species coding for ZFNs

For the modification of HEK293 cells, expression vectors encoding HLA-A targeting ZFNs were introduced by nucleofection (Lonza) using the manufacturer's protocol. T cells were harvested 6 days after initial stimulation or 2 to 3 days after re-stimulation with γ-irradiated aAPC. Five million T cells were premixed with 2.5 to 10 µg of each ZFN-L and ZFN-R mRNA species in 100 µL of Human T Cell Nucleofector solution (Lonza) and electroporated in a cuvette using a Nucleofector II device with program T-20. Following electroporation, cells were immediately placed in pre-warmed RPMI 1640 supplemented with 2 mmol/L L-glutamine and 10% FBS, and cultured at 37°C and 5% CO2 for 4-6 hours, at which point 50 IU/mL of IL-2 was added for further culture. In "cold shock" experiments, after overnight culture in a 37°C-5% CO2 incubator, T cells were transferred to 30°C, 5% CO2 incubator and cultured for 3 days, and then returned to a 37°C, 5% CO2 incubator prior to analysis.

### Enrichment of cells lacking expression of HLA-A

After washing cells with phosphate buffered saline (PBS) supplemented with 2% FBS and 2mM EDTA, cells were labeled with PE-conjugated monoclonal antibody (mAb) specific anti-HLA-A2 (BD Biosciences, San Jose, CA) at 4°C for 15 minutes, washed, and labeled with anti-PE microbeads (Miltenyi Biotec, Auburn, CA) for 10 minutes. After washing, labeled cells were passed through an LD column (MiltenyiBiotec) and the flow-through fraction was collected and cultured. T cells were propagated on γ-irradiated OKT3-loaded aAPC and CAR+ T cells were propagated on CD19+ aAPC (not OKT3-loaded) in RPMI 1640 supplemented with 2 mmol/L L-glutamine and 10% FBS with 50 IU/mL of IL-2 (added every other day).

### Flow cytometry

The following antibodies were used: phycoerythrin (PE) anti HLA-A2 (clone BB7.2), FITC anti-CD4 (clone RPA-T4), FITC anti-CD8 (clone HIT8a), PE and APC anti-CD3 (clone SK7), PE anti-CD56 (clone B159), PE anti HLA-DR (clone G46-6), PE-mouse IgG2by, PE mouse IgG2ak, FITC non-specific mouse IgG1, secondary reagent streptavidin-PE (all from BD Biosciences), biotin-conjugated anti-HLA-A3(clone 4i153), APC anti-HLA-G (clone MEMG19), PE anti HLA class I (clone W6/32, from Abcam, Cambridge, MA), and PE anti-HLA-E (clone 3D12, Biolegend, San Diego, CA). The Alexa 488-conjugated anti-CD19RCD28 CAR antibody (clone no. 136-20-1) was generated in our laboratory. We added propidium iodide (Sigma-Aldrich) to exclude dead cells from analysis. Data was acquired on a FACS Calibur (BD Biosciences) using CellQuest version 3.3 (BD Biosciences) and analyzed by FlowJo version 7.6.1 (Tree Star, Inc. Ashland, OR).

### Surveyor™ nuclease assay

The level of modification of the HLA-A gene sequence in ZFN transfected cells was determined by the Surveyor™ nuclease assay as described in Guschin et al. (2010) Methods Mol Biol 649:247-256. In brief, genomic DNA from ZFN-modified cells underwent PCR with oligonucleotide primers designed to amplify the ZFN target regions within HLA-A2 and HLA-A3 genetic loci. After denaturing and re-annealing, Surveyor endonuclease (Cel-1) (Transgenomic, Omaha, NE) was used to cut heteroduplex DNA products to reveal a fast-moving band on polyacrylamide gel that was interpreted as evidence of a mutation event. Percent target modification was quantified by densitometry. The PCR primers used for the amplification of target loci were;
HLA-A3 Forward; 5'- GGGGCCGGAGTATTGGGACCA -3'; (SEQ ID NO:7) HLA-A3 Reverse; 5'- CCGTCGTAGGCGTCCTGCCG -3' (SEQ ID NO:8) HLA-A2 Forward; 5'- GGGTCCGGAGTATTGGGACGG-3' (SEQ ID NO:9) HLA-A2 Reverse; 5'- TTGCCGTCGTAGGCGTACTGGTG -3' (SEQ ID NO:10) HLA-A2 and HLA-A3 sequences were obtained from IMGT/HLA database, for example IMGT/HLA Accession no.; HLA-A2:HLA00005, HLA-A3: HLA00037.

### ⁵¹Chromium release assay (CRA)

Target cells were labeled with 0.1 mCi of ⁵¹Cr for 2 hours. After washing thrice with ice-cold RPMI 1640 supplemented with 10% FBS, labeled cells were diluted and distributed at 10³ target cells in 100 µL per well in 96-well, v-bottomed plates. In the peptide titration assay, target cells were incubated with 10-fold serial dilutions of the peptides for 30 minutes at room temperature. CTL were added at indicated effector to target ratios. After 4-hour incubation at 37°C, in 5% CO2, 50 µL of cell-free supernatants were collected and counted on a TopCount device (Perkin Elmer, Shelton, CT). All assays were performed in triplicate. In some assays, parental HEK293 cell lines and HLA-A modified HEK293 clones were treated with 600 IU/mL of interferon-γ (IFN-γ; R&D systems, Minneapolis, MN) and 10 ng/mL of tissue necrosis factor-α (TNF-α; R&D systems) for 48 hours before assay. The percent specific lysis was calculated as follows: ((experimental cpm - spontaneous cpm) / (maximum cpm - spontaneous cpm)) × 100.

### NK-cell isolation and enforced expression of non-classical HLA on 721.221 cells

NK cells were isolated from PBMC by CD56 microbeads (Miltenyi Biotec) and LS columns (Miltenyi Biotec) according to the manufacture's instruction. DNA plasmids coding for SB transposons HLA-E (accession no. 005516) and/or HLA-G (accession no. NM_002127) were co-electroporated with SB11 transposase into parental HLA class I^{low} 721.221 cells by Amaxa Nucleofector II device (program: A-016). HLA-E+ and HLA G+ clones exhibiting stable and homogeneous expression of introduced HLA molecules were derived by limiting dilution after sorting HLA-E and/or HLA-G positive cells by fluorescence-conjugated mAbs [PE anti-HLA-E, APC anti-HLA-G, and PE anti-HLA-G (clone 87G, Biolegend)] and paramagnetic beads [anti PE microbeads and anti APC microbeads (cat #s 130-048-801, 130-090-855 (Miltenyi Biotec)]. NK cell killing of 721.221 clones was assessed by 4-hr CRA and statistical differences of the data were calculated by one-way ANOVA followed by Tukey's multiple comparison in GraphPad Prism software (version 5, GraphPad Software, La Jolla, CA).

### Culture and differentiation of hESC

The hESC line WIBR3 (Whitehead Institute Center for Human Stem Cell Research, Cambridge, MA) 22 was maintained as described previously (Soldner et al. (2009) Cell 136(5):964-977 on mitomycin C inactivated mouse embryonic fibroblast (MEF) feeder layers in hESC medium [DMEM/F12 (Invitrogen) supplemented with 15% FBS, 5% KnockOut™ Serum Replacement (Invitrogen), 1 mM glutamine (Invitrogen), 1% nonessential amino acids (Invitrogen), 0.1 mM β-mercaptoethanol (Sigma, St. Louis, MO) and 4 ng/ml FGF2 (R&D systems)]. Targeted hESC were differentiated into fibroblast-like cells as described previously (Hockemeyer et al. (2008) Cell Stem Cell 3(3):346-353. Briefly, differentiation was induced by embryoid body (EB) formation in non-adherent suspension culture dishes (Corning, Corning, NY) in DMEM medium supplemented with 15% fetal bovine serum for 5 days. EBs were subsequently plated onto adherent tissue culture dishes and passaged according to primary fibroblast protocols using trypsin for at least four passages before the start of experiments.

### ZFN-mediated genome editing of hESC

HESC were cultured in Rho-associated protein kinase (ROCK)-inhibitor (Stemolecule; Stemgent, Cambridge, MA) 24 hours prior to electroporation. Cells were harvested using 0.05% trypsin/EDTA solution (Invitrogen) and resuspended in PBS. Ten million cells were electroporated (Gene Pulser Xcell System, Bio-Rad: 250 V, 500µ F, 0.4 cm cuvettes) with 35 µg of donor plasmid encoding puromycin resistant gene under control of phosphoglycerate kinase (PGK) promoter flanked by 5' and 3' arms homologous to the putative ZFN binding region of HLA-A24 and 7.5 µg of each ZFN-encoding plasmid, or 35 µg of donor plasmid and 10 µg of each ZFN encoding mRNA. Cells were subsequently plated on DR4 MEF feeder layers in hESC medium supplemented with ROCK inhibitor for the first 24 hours. Puromycin selection (0.5 µg/ml) was initiated 72 hours after electroporation. Individual puromycin-resistant colonies were picked and expanded 10 to 14 days after electroporation. Correct targeting and gene disruption was verified by Southern blot analysis and sequencing of the genomic locus.

### Example 2: Design and Validation of Zinc Finger Nucleases Targeting Multiple endogenous HLA-A Genes

ZFNs were designed to cleave a pre-defined site within the genomic coding sequence of the endogenous human HLA-A genes (see, *e.g.,* Table 2 of U.S. Patent Publication 2012/0060230). Expression of these ZFNs in human cells should eliminate expression of HLA-A molecules via error-prone repair of introduced double-strand breaks leading to disruption of the reading frame of the targeted HLA loci. To evaluate the ability of these ZFNs to disrupt HLA-A expression we initially used the human embryonic kidney cell line HEK293, which co-expresses HLA-A*03:01 (HLA-A3) and HLA-A*02:01 (HLA-A2). After transfecting HEK293 cells with expression plasmids encoding the ZFNs as described in Example 1, we used allele-specific PCR and the Surveyor nuclease assay to quantify the level of gene modification at the anticipated ZFN target sites.

As shown in Figure 1, approximately 10% modification of HLA-A3 locus and ∼6% modification of HLA-A2 locus were modified HLA-A targeted ZFNs.

### Example 3: Isolation and Functional Validation of HLA-A^{neg} HEK293 Cells

To assess the impact of disrupting HLA-A expression, we used limiting dilution to obtain single-cell clones from the ZFN-modified HEK293 cell pool. Sequencing revealed clones that carried small insertions or deletions within the expected ZFN-binding sites in HLA-A2, HLA-A3, or both alleles, which resulted in a frame shift leading to premature termination of translation. Since the steady state level of HLA-A expression in HEK293 cells is low compared with hematopoietic cells, such as an EBV transformed lymphoblastoid cell line (EBV-LCL), we exposed the HEK293 cells to pro-inflammatory cytokines known to augment HLA levels. See, *e.g.,* Johnson (2003) J Immunol. 170(4):1894-1902.

The addition of interferon-gamma (IFN-γ) and tissue-necrosis-factor-alpha (TNF-α) increased expression of both HLA-A2 and HLA-A3 in parental HEK293 cells (Fig. 2A, top panel). In contrast, ZFN-treated HEK293 clones carrying mutations in HLA-A2 and/or HLA-A3 did not express these proteins even after induction by IFN-γ and TNF-α (Fig. 2A, bottom 3 panels). Thus, flow cytometry using mAbs specific for HLA-A2 or HLA-A3 confirmed the allele-specific loss of HLA-A expression on the cell surface.

Next, we asked whether the loss of HLA-A expression on the ZFN-modified clones would preclude T-cell recognition and this was tested using HLA-A3 and HLA-A2-restricted cytotoxic T-lymphocyte (CTL) clones. As expected, an HLA-A3-restricted CD8+ CTL clone 7A7 demonstrated robust specific lysis of the HLA-A3+ parental HEK293 cells loaded with serial dilutions of cognate peptide (RVWDLPGVLK, SEQ ID NO:1, NP_001103685) with 50% maximal lysis observed with 1 ng/mL of the pulsed cognate peptide (Fig. 2B, top panel). HEK293 clone 8.18 that has lost expression of HLA-A2 allele, but is wild type at HLA-A3, was also lysed by this HLA-A3 restricted CTL clone. In contrast, when pulsed with the same peptide, the HEK293 clone 18.1 that had been edited to eliminate HLA-A3 expression, was not lysed by the HLA-A3 restricted CTL clone 7A7, and neither was the HLA-A2/A3 double-knock out HEK293 clone 83 (Fig.2B, top panel). We also evaluated the cytolytic activity of an HLA-A2 restricted CTL clone GAS2B3-5 and observed robust killing activity when presented with the parental HEK293 cells or the HLA-A2 wild type clone 18.1, while the ZFN-modified HLA-A2^{neg} HEK293 clone 8.18 and the HLA-A2/A3 double-knock out clone 83 were spared from lysis (Fig. 2B, bottom panel).

These data demonstrate that treatment with ZFNs completely eliminates HLA-A expression, resulting in protection from HLA-A restricted CTL-mediated killing, even under pro-inflammatory conditions that up-regulate endogenous HLA-A expression.

### Example 4: NK-cell mediated lysis against HLA^{null} cells can be prevented by enforced expression of HLA-E and/or HLA-G

We envision that the approach we outline here, using ZFN targeting HLA class I genes combined with antibody based cell sorting, could ultimately be used to eliminate expression of HLA-A, -B and -C expression. Cells without classical HLA expression, especially HLA-B or HLA-C which are known to be the main ligands for killer inhibitory receptors (KIRs), may be eradicated through the loss of interaction between KIR and its ligand. Parham et al. (2005) Nat Rev Immunol. 5(3):201-214. To test whether NK-cell mediated cytoxicity would be reduced, we introduced non-classical HLA-E or HLA-G molecules, which have been shown to reduce NK-cell mediated cytotoxicity (Borrego et al. (1998) J Exp Med. 187(5):813-818; Riteau et al. (2001) Int Immunol. 13(2):193-201; Rouas-Freiss et al. (1997) Proc Natl Acad Sci U S A. 94(10):5249-5254; Braud et al. (1998) Nature 391(6669):795-799) and are much less polymorphic than classical HLA molecules, into the HLA class Ilow cell line 721.221 (Fig. 3A) and evaluated their susceptibility to be killed by NK cells.

The flow cytometry analysis of NK cells directly isolated from PBMC showed over 94% purity (CD56^{pos}CD3^{neg} population) (Fig. 4A) and HLA-E and/or HLA-G expression in genetically modified 721.221 clones at over 90% (Fig. 4B). We demonstrated that enforced expression of HLA-E and/or HLA-G on 721.221 significantly prevented these target cells from NK-cell mediated lysis (Fig. 4C).

This provides a solution to forestall elimination of administered HLA^{neg} allogeneic cells by recipient NK cells, thus rendering the complete HLA class I knock out feasible for human application by avoiding the introduction of immunogenic transgenes.

### Example 5: Disruption of HLA-A Genes in Primary T cells using a "Hit-and-Run" Strategy

To extend our results to clinically relevant primary cells, we evaluated the activity of the HLA-A-specific ZFNs in human T cells. Since ZFNs require only temporary expression to achieve stable disruption of desired target genes, we transiently expressed ZFNs from an in vitro transcribed mRNA. Electro-transfer of mRNA encoding the ZFNs into PBMC from an HLA-A2 homozygous donor (HLA-A2 being the most common HLA-A allele in Caucasians, see, e.g., Mori et al. (1997) Transplantation 64(7):1017-1027) rendered ∼19% of these T cells HLA-A2 negative (Fig. 5A, top panel). We have previously demonstrated that transiently lowering the incubation temperature after transfection can increase ZFN activity. See, U.S. Patent Publication No. 2011/0129898.

Subjecting electroporated primary T cells to a transient hypothermia elevated the proportion of HLA-A2 negative cells by up to 57% in an mRNA dose dependent manner (Fig. 5A, bottom panel).

### Example 6: Achieving a Clinically Relevant Level of HLA-A Disruption in Primary T Cells

With a view to the clinical application of the HLA-targeted ZFNs, we evaluated the use of the "high fidelity" obligate heterodimeric Fok I domains EL/KK, which are designed to decrease potential off-target cleavage events by preventing homodimerization33. Use of mRNA encoding the EL/KK ZFN variants of ZFN-L and ZFN-R resulted in an marked increase in HLA-A^{neg} T cells, eliminating HLA-A expression in up to 52% of T-cell population, despite limiting doses of mRNA (2.5 µg each ZFN) (Fig. 5B).

A single round of HLA-A positive T cell depletion with antibody-coated paramagnetic beads readily increased the HLA-A2^{neg} T-cell fraction to over 95% of the population without impacting CD4 or CD8 expression (Fig. 6A). Analysis of this HLA-A2^{neg} population by the Surveyor nuclease assay (Fig. 6B) and direct DNA sequencing (Fig. 6C) revealed nearly 100% editing of the HLA-A2 alleles precisely within the region targeted by the ZFNs.

Together these data demonstrate that ZFN-driven genome editing can rapidly generate an HLA-negative primary T cell population.

### Example 7: Disruption of HLA-A Genes in T cells Genetically Modified to Redirect Specificity

To demonstrate the potential utility of HLA editing, we next focused on a specific class of cells that could be broadly used in allogeneic settings after elimination of HLA expression; namely cytotoxic T cells genetically modified to express a 'universal' chimeric antigen receptor (CAR) to redirect specificity towards tumor associated antigens independent of HLA recognition 34. Indeed, we and others are currently infusing patient-specific CAR+ T cells for the investigational treatment of CD19+ malignancies. (*See, e.g.,* Kalos et al. (2011) Sci Transl Med 3(95):95ra73; Porter et al. (2011) N Engl J Med 365(8):725-733). Recently, we have published that CAR+ T cells retain redirected specificity for CD 19 when ZFNs are used to eliminate endogenous αβTCR expression (Torikai et al. (2012) Blood 119(24):5697-5705 and Provasi et al, (2012) Nat Med 18(5):807-15). Indeed, such TCR-edited T cells demonstrate both improved potency and safety (GVHD) *in vivo.*

To further our ability to generate "off-the-shelf" T cell therapies, we investigated whether ZFNs could eliminate HLA-A expression from primary T cells previously engineered to express a CD19-specific CAR. PBMC genetically modified by synchronous electro-transfer of DNA plasmids derived from the Sleeping Beauty (SB) transposon/transposase system followed by selective propagation on CD19+ aAPC, clone #439 resulted in expression of the CD19-specific CAR (designated CD19RCD28) in over 90% of the T cells. These SB and aAPC platforms have been adapted by us for human application in four clinical trials (INDs #14193, 14577, 14739, and 15180).

CAR+ T cells were subsequently electroporated with *in vitro-*transcribed mRNA encoding the obligate heterodimeric variants of the HLA-A ZFNs. ZFN treatment successfully disrupted HLA-A2 expression in ∼22% of CAR+ T cells without selection, and this population was readily enriched to ∼99% HLA-A2^{neg} cells by negative selection for HLA-positive cells (Fig. 6A). These cells were shown to maintain their new phenotype since after 50 days of continuous co-culture on CD19+ aAPC ∼94% of the CAR+ T cells remained HLA-A2^{neg}. Importantly, these HLA-A2^{neg} T cells evaded attack by HLA-A2 restricted CTLs (Fig. 6B), and maintained their anti-tumor activity as evidenced by CAR-dependent lysis of CD19+ tumor targets (Fig. 6C).

In aggregate, these data demonstrate that CAR redirected tumor specific T cells can be genetically modified by ZFNs to eliminate HLA-A expression. Such HLA-A^{neg} cells have the potential to enable "off the shelf' tumor-specific T cells that can be pre-prepared from one donor and infused on demand into multiple recipients.

### Example 8: Disruption of the HLA-A Gene in hESC

To broaden the application of allogeneic cells for therapeutic applications, including tissue regeneration, we sought to generate hESC capable of evading T-cell recognition. By definition, all hESC are allogeneic with respect to potential recipients and upon differentiation will upregulate expression of HLAs40. To test the use of ZFNs for the generation of HLA-A^{neg} hESC, we genetically modified the HLA-A2+/A24+ hESC line WIBR3 with either mRNA or DNA plasmids encoding ZFNs targeting HLA-A loci. To facilitate generation of HLA-A^{neg} hESC we co-delivered a donor DNA plasmid encoding the puromycin resistance gene flanked by regions of homology surrounding the ZFN target site to mediate targeted integration by homology-directed repair. Puromycin-resistant clones were screened for modification of the HLA-A alleles by PCR sequencing of the ZFN target region and by Southern Blot analysis of the targeted region using probes located outside of the donor homology arms. There clones were containing mutations in the ZFN target region in both HLA-A alleles, and differentiated into fibroblast-like cells along with the unmodified parental hESC line. HLA expression was induced by treatment with IFN-γ and TNF-α and analyzed by flow cytometry with antibodies recognizing HLA-A2 and HLA-A24, respectively.

While the parental cell line exhibited strong expression of both HLA alleles, all 3 knockout lines lacked cell surface expression of both HLA-A alleles (Fig. 7). These data demonstrate the portability of the HLA-A knockout approach to hESCs - which may be a necessary step for cell persistence post-transplantation.

## Claims

1. An isolated Natural Killer (NK) cell comprising one or more non-classic class I human leukocyte antigen (HLA) proteins and further wherein at least one classic endogenous HLA gene within the cell is inactivated by a zinc finger nuclease.

2. The NK cell of claim 1, wherein the non-classic class I HLA proteins are selected from the group consisting of HLA-E, HLA-F, HLA-G and combinations thereof.

3. The NK cell of claim 1 or claim 2, wherein the non-classic class I HLA proteins are expressed from endogenous genes.

4. The NK cell of any one of claims 1 to 3, wherein the non-classic class I HLA proteins are expressed from exogenous sequences.

5. The NK cell of any one of claims 1 to 4, wherein the zinc finger nuclease comprises a zinc finger protein comprising the recognition helix regions as shown in a single row of the following table:
| Target | SBS # | Design | | | | | |
|---|---|---|---|---|---|---|---|
| **Class I** | | F1 | F2 | F3 | F4 | F5 | F6 |
| **HLA A2** | 18889 | QSSHLTR (SEQ ID NO:11) | RSDHLTT (SEQ ID NO:12) | RSDTLSQ (SEQ ID NO:13) | RSADLSR (SEQ ID NO:14) | QSSDLSR (SEQ ID NO:15) | RSDALTQ (SEQ ID NO:16) |
| **HLA A2** | 18881 | QKTHLAK (SEQ ID NO:17) | RSDTLSN (SEQ ID NO:18) | RKDVRIT (SEQ ID NO:19) | RSDHLST (SEQ ID NO:20) | DSSARKK (SEQ ID NO:21) | NA |
| **HLA A2** | 24859 | QNAHRKT (SEQ ID NO:22) | RSDSLLR (SEQ ID NO:23) | RNDDRKK (SEQ ID NO:24) | RSDHLST (SEQ ID NO:20) | DSSARKK (SEQ ID NO:21) | NA |
| **HLA A3** | 25191 | DRSHLSR (SEQ ID NO:25) | RSDDLTR (SEQ ID NO:26) | DRSDLSR (SEQ ID NO:27) | QSGHLSR (SEQ ID NO:28) | NA | NA |
| **HLA A3** | 25190 | DRSALSR (SEQ ID NO:29) | QSSDLRR (SEQ ID NO:30) | DRSALSR (SEQ ID NO:29) | DRSHLAR (SEQ ID NO:31) | RSDDLSK (SEQ ID NO:32) | DRSHLAR (SEQ ID NO:31) |

6. The NK cell of any one of claims 1 to 5, wherein the cell comprises one or more additional genomic modifications.

7. A pharmaceutical composition comprising the NK cell of any one of claims 1 to 6.

8. The pharmaceutical composition of claim 7 for use in a method of treating graft-versus-host disease, Addison's disease, ankylosing spondylitis, Behçet's disease, Buerger's disease, celiac disease, chronic active hepatitis, Graves' disease, juvenile rheumatoid arthritis, psoriasis, psoriatic arthritis, rheumatoid arthritis, Sjogren syndrome, or lupus erythematosus in a subject in need thereof, the method comprising administering a NK cell according to any one of claims 1 to 6 to the subject.

9. The pharmaceutical composition for use according to claim 8, wherein the HLA-related disorder is graft-versus-host disease (GVHD).

10. A method of generating an isolated Natural Killer (NK) cell in which one or more classic HLA genes are inactivated and in which one or more non-classic HLA proteins are present within the cell, the method comprising;
a) cleaving an endogenous HLA gene or HLA regulator gene in said cell with a zinc finger nuclease, such that the HLA or HLA regulator gene is inactivated, and;
b) introducing a nucleic acid encoding a non-classic HLA molecule into the cell.

11. The method of generating an isolated NK cell of claim 10, wherein the zinc finger nuclease comprises a zinc finger protein comprising the recognition helix regions as shown in a single row of the following table:
| Target | SBS # | Design | | | | | |
|---|---|---|---|---|---|---|---|
| **Class I** | | F1 | F2 | F3 | F4 | F5 | F6 |
| **HLA A2** | 18889 | QSSHLTR (SEQ ID NO:11) | RSDHLTT (SEQ ID NO:12) | RSDTLSQ (SEQ ID NO:13) | RSADLSR (SEQ ID NO:14) | QSSDLSR (SEQ ID NO:15) | RSDALTQ (SEQ ID NO:16) |
| **HLA A2** | 18881 | QKTHLAK (SEQ ID NO:17) | RSDTLSN (SEQ ID NO:18) | RKDVRIT (SEQ ID NO:19) | RSDHLST (SEQ ID NO:20) | DSSARKK (SEQ ID NO:21) | NA |
| **HLA A2** | 24859 | QNAHRKT (SEQ ID NO:22) | RSDSLLR (SEQ ID NO:23) | RNDDRKK (SEQ ID NO:24) | RSDHLST (SEQ ID NO:20) | DSSARKK (SEQ ID NO:21) | NA |
| **HLA A3** | 25191 | DRSHLSR (SEQ ID NO:25) | RSDDLTR (SEQ ID NO:26) | DRSDLSR (SEQ ID NO:27) | QSGHLSR (SEQ ID NO:28) | NA | NA |
| **HLA A3** | 25190 | DRSALSR (SEQ ID NO:29) | QSSDLRR (SEQ ID NO:30) | DRSALSR (SEQ ID NO:29) | DRSHLAR (SEQ ID NO:31) | RSDDLSK (SEQ ID NO:32) | DRSHLAR (SEQ ID NO:31) |

## Patentansprüche

1. Isolierte natürliche Killerzelle (NK-Zelle), die ein oder mehrere nichtklassische menschliche Leukozytenantigen- (HLA-) Proteine der Klasse I umfasst und wobei außerdem zumindest ein klassisches endogenes HLA-Gen innerhalb der Zelle durch eine Zinkfingernuclease inaktiviert ist.

2. NK-Zelle nach Anspruch 1, wobei die nichtklassischen HLA-Proteine der Klasse I aus der aus HLA-E, HLA-F, HLA-G und Kombinationen davon bestehenden Gruppe ausgewählt sind.

3. NK-Zelle nach Anspruch 1 oder Anspruch 2, wobei die nichtklassischen HLA-Proteine der Klasse I aus endogenen Genen exprimiert sind.

4. NK-Zelle nach einem der Ansprüche 1 bis 3, wobei die nichtklassischen HLA-Proteine der Klasse I aus exogenen Sequenzen exprimiert sind.

5. NK-Zelle nach einem der Ansprüche 1 bis 4, wobei die Zinkfingernuclease ein Zinkfingerprotein umfasst, das die Erkennungshelixregionen umfasst, wie sie in einer einzelnen Zeile der folgenden Tabelle dargestellt sind:
| Target | **SBS Nr.** | Design | | | | | |
|---|---|---|---|---|---|---|---|
| **Klasse I** | | F1 | F2 | F3 | F4 | F5 | F6 |
| **HLA A2** | 18889 | QSSHLTR (SEQ-ID Nr.11) | RSDHLTT (SEQ-ID Nr.12) | RSDTLSQ (SEQ-ID Nr.13) | RSADLSR (SEQ-ID Nr.14) | QSSDLSR (SEQ-ID Nr.15) | RSDALTQ (SEQ-ID Nr.16) |
| **HLA A2** | 18881 | QKTHLAK (SEQ-ID Nr.17) | RSDTLSN (SEQ-ID Nr.18) | RKDVRIT (SEQ-ID Nr.19) | RSDHLST (SEQ-ID Nr.20) | DSSARKK (SEQ-ID Nr.21) | k.A. |
| **HLA A2** | 24859 | QNAHRKT (SEQ-ID Nr.22) | RSDSLLR (SEQ-ID Nr.23) | RNDDRKK (SEQ-ID Nr.24) | RSDHLST (SEQ-ID Nr.20) | DSSARKK (SEQ-ID Nr.21) | k.A. |
| **HLA A3** | 25191 | DRSHLSR (SEQ-ID Nr.25) | RSDDLTR (SEQ-ID Nr.26) | DRSDLSR (SEQ-ID Nr.27) | QSGHLSR (SEQ-ID Nr.28) | k.A. | k.A. |
| **HLA A3** | 25190 | DRSALSR (SEQ-ID Nr.29) | QSSDLRR (SEQ-ID Nr.30) | DRSALSR (SEQ-ID Nr.29) | DRSHLAR (SEQ-ID Nr.31) | RSDDLSK (SEQ-ID Nr.32) | DRSHLAR (SEQ-ID Nr.31) |

6. NK-Zelle nach einem der Ansprüche 1 bis 5, wobei die Zelle eine oder mehrere zusätzliche genomische Modifikationen aufweist.

7. Pharmazeutische Zusammensetzung, die eine NK-Zelle nach einem der Ansprüche 1 bis 6 umfasst.

8. Pharmazeutische Zusammensetzung nach Anspruch 7 zur Verwendung in einem Verfahren zur Behandlung von Transplantat-gegen-Wirt-Krankheit, Addison-Krankheit, ankylosierender Spondylitis, Behçet-Krankheit, Buerger-Krankheit, Zöliakie, chronischer aktiver Hepatitis, Basedowscher Krankheit, juveniler rheumatoider Arthritis, Psoriasis, Psoriaris-Arthritis, rheumatoider Arthritis, Sjögren-Syndrom oder Lupus erythematodes bei einem Individuum, das dies benötigt, wobei das Verfahren die Verabreichung einer NK-Zelle nach einem der Ansprüche 1 bis 6 an das Individuum umfasst.

9. Pharmazeutische Zusammensetzung zur Verwendung nach Anspruch 8, wobei die mit HLA zusammenhängende Störung Transplantat-gegen-Wirt-Krankheit (GVHD) ist.

10. Verfahren zur Herstellung einer isolierten natürlichen Killerzelle (NK-Zelle), in der ein oder mehr klassische HLA-Gene inaktiviert sind und in der ein oder mehr nichtklassische HLA-Proteine in der Zelle vorhanden sind, wobei das Verfahren Folgendes umfasst:
a) Spalten eines endogenen HLA-Gens oder eines HLA-Regulatorgens in der Zelle mit einer Zinkfingernuclease, sodass das HLA- oder HLA-Regulatorgen inaktiviert wird; und
b) Einführen einer Nucleinsäure, die für ein nichtklassisches HLA-Molekül kodiert, in die Zelle.

11. Verfahren zur Herstellung einer isolierten NK-Zelle nach Anspruch 10, wobei die Zinkfingernuclease ein Zinkfingerprotein umfasst, das die Erkennungshelixregionen umfasst, wie sie in einer einzelnen Zeile der folgenden Tabelle dargestellt sind:
| Target | SBS Nr. | Design | | | | | |
|---|---|---|---|---|---|---|---|
| Klasse I | | F1 | F2 | F3 | F4 | F5 | F6 |
| **HLA A2** | 18889 | QSSHLTR (SEQ-ID Nr.11) | RSDHLTT (SEQ-ID Nr.12) | RSDTLSQ (SEQ-ID Nr.13) | RSADLSR (SEQ-ID Nr.14) | QSSDLSR (SEQ-ID Nr.15) | RSDALTQ (SEQ-ID Nr.16) |
| **HLA A2** | 18881 | QKTHLAK (SEQ-ID Nr.17) | RSDTLSN (SEQ-ID Nr.18) | RKDVRIT (SEQ-ID Nr.19) | RSDHLST (SEQ-ID Nr.20) | DSSARKK (SEQ-ID Nr.21) | k.A. |
| **HLA A2** | 24859 | QNAHRKT (SEQ-ID Nr.22) | RSDSLLR (SEQ-ID Nr.23) | RNDDRKK (SEQ-ID Nr.24) | RSDHLST (SEQ-ID Nr.20) | DSSARKK (SEQ-ID Nr.21) | k.A. |
| **HLA A3** | 25191 | DRSHLSR (SEQ-ID Nr.25) | RSDDLTR (SEQ-ID Nr.26) | DRSDLSR (SEQ-ID Nr.27) | QSGHLSR (SEQ-ID Nr.28) | k.A. | k.A. |
| **HLA A3** | 25190 | DRSALSR (SEQ-ID Nr.29) | QSSDLRR (SEQ-ID Nr.30) | DRSALSR (SEQ-ID Nr.29) | DRSHLAR (SEQ-ID Nr.31) | RSDDLSK (SEQ-ID Nr.32) | DRSHLAR (SEQ-ID Nr.31) |

## Revendications

1. Cellule tueuse naturelle (NK) isolée comprenant une ou plusieurs protéines d'antigène des leucocytes humains (HLA) de classe I non classiques et en outre dans laquelle au moins un gène HLA endogène classique au sein de la cellule est inactivé par une nucléase à doigts de zinc.

2. Cellule NK selon la revendication 1, dans laquelle les protéines HLA de classe I non classiques sont sélectionnées dans le groupe consistant en HLA-E, HLA-F, HLA-G et des combinaisons de celles-ci.

3. Cellule NK selon la revendication 1 ou la revendication 2, dans laquelle les protéines HLA de classe I non classiques sont exprimées à partir de gènes endogènes.

4. Cellule NK selon l'une quelconque des revendications 1 à 3, dans laquelle les protéines HLA de classe I non classiques sont exprimées à partir de séquences exogènes.

5. Cellule NK selon l'une quelconque des revendications 1 à 4, dans laquelle la nucléase à doigts de zinc comprend une protéine à doigts de zinc comprenant les régions d'hélice de reconnaissance telles que montrées dans une seule ligne du tableau suivant :
| Cible | SBS n° | Conception | | | | | |
|---|---|---|---|---|---|---|---|
| **Classe I** | | F1 | F2 | F3 | F4 | F5 | F6 |
| **HLA A2** | 18889 | QSSHLTR (SEQ ID NO: 11) | RSDHLTT (SEQ ID NO: 12) | RSDTLSQ (SEQ ID NO: 13) | RSADLSR (SEQ ID NO: 14) | QSSDLSR (SEQ ID NO: 15) | RSDALTQ (SEQ ID NO: 16) |
| **HLA A2** | 18881 | QKTHLAK (SEQ ID NO: 17) | RSDTLSN (SEQ ID NO: 18) | RKDVRIT (SEQ ID NO: 19) | RSDHLST (SEQ ID NO: 20) | DSSARKK (SEQ ID NO: 21) | NA |
| **HLA A2** | 24859 | QNAHRKT (SEQ ID NO: 22) | RSDSLLR (SEQ ID NO: 23) | RNDDRKK (SEQ ID NO: 24) | RSDHLST (SEQ ID NO: 20) | DSSARKK (SEQ ID NO: 21) | NA |
| **HLA A3** | 25191 | DRSHLSR (SEQ ID NO: 25) | RSDDLTR (SEQ ID NO: 26) | DRSDLSR (SEQ ID NO: 27) | QSGHLSR (SEQ ID NO: 28) | NA | NA |
| **HLA A3** | 25190 | DRSALSR (SEQ ID NO: 29) | QSSDLRR (SEQ ID NO: 30) | DRSALSR (SEQ ID NO: 29) | DRSHLAR (SEQ ID NO: 31) | RSDDLSK (SEQ ID NO: 32) | DRSHLAR (SEQ ID NO: 31) |

6. Cellule NK selon l'une quelconque des revendications 1 à 5, où la cellule comprend une ou plusieurs modifications génomiques supplémentaires.

7. Composition pharmaceutique comprenant la cellule NK selon l'une quelconque des revendications 1 à 6.

8. Composition pharmaceutique selon la revendication 7 destinée à être utilisée dans un procédé de traitement de la maladie du greffon contre l'hôte, la maladie d'Addison, la spondylarthrite ankylosante, la maladie de Behçet, la maladie de Buerger, la maladie coeliaque, une hépatite chronique active, la maladie de Graves, la polyarthrite rhumatoïde juvénile, le psoriasis, l'arthrite psoriasique, la polyarthrite rhumatoïde, le syndrome de Sjögren, ou le lupus érythémateux chez un sujet en ayant besoin, le procédé comprenant l'administration d'une cellule NK selon l'une quelconque des revendications 1 à 6 au sujet.

9. Composition pharmaceutique destinée à être utilisée selon la revendication 8, dans laquelle le trouble lié à HLA est la maladie du greffon contre l'hôte (MGCH).

10. Procédé de génération d'une cellule tueuse naturelle (NK) isolée dans laquelle un ou plusieurs gènes HLA classiques sont inactivés et où une ou plusieurs protéines HLA non classiques sont présentes au sein de la cellule, le procédé comprenant :
a) le clivage d'un gène HLA endogène ou d'un gène de régulation HLA dans ladite cellule avec une nucléase à doigts de zinc, de sorte que le gène HLA ou de régulation HLA soit inactivé, et ;
b) l'introduction d'un acide nucléique codant pour une molécule HLA non classique dans la cellule.

11. Procédé de génération d'une cellule NK isolée selon la revendication 10, dans lequel la nucléase à doigts de zinc comprend une protéine à doigts de zinc comprenant les régions d'hélice de reconnaissance telles que montrées dans une seule ligne du tableau suivant :
| Cible | SBS n° | Conception | | | | | |
|---|---|---|---|---|---|---|---|
| **Classe I** | | F1 | F2 | F3 | F4 | F5 | F6 |
| **HLA A2** | 18889 | QSSHLTR (SEQ ID NO: 11) | RSDHLTT (SEQ ID NO: 12) | RSDTLSQ (SEQ ID NO: 13) | RSADLSR (SEQ ID NO: 14) | QSSDLSR (SEQ ID NO: 15) | RSDALTQ (SEQ ID NO: 16) |
| **HLA A2** | 18881 | QKTHLAK (SEQ ID NO: 17) | RSDTLSN (SEQ ID NO: 18) | RKDVRIT (SEQ ID NO: 19) | RSDHLST (SEQ ID NO: 20) | DSSARKK (SEQ ID NO: 21) | NA |
| **HLA A2** | 24859 | QNAHRKT (SEQ ID NO: 22) | RSDSLLR (SEQ ID NO: 23) | RNDDRKK (SEQ ID NO: 24) | RSDHLST (SEQ ID NO: 20) | DSSARKK (SEQ ID NO: 21) | NA |
| **HLA A3** | 25191 | DRSHLSR (SEQ ID NO: 25) | RSDDLTR (SEQ ID NO: 26) | DRSDLSR (SEQ ID NO: 27) | QSGHLSR (SEQ ID NO: 28) | NA | NA |
| **HLA A3** | 25190 | DRSALSR (SEQ ID NO: 29) | QSSDLRR (SEQ ID NO: 30) | DRSALSR (SEQ ID NO: 29) | DRSHLAR (SEQ ID NO: 31) | RSDDLSK (SEQ ID NO: 32) | DRSHLAR (SEQ ID NO: 31) |
